(19) Europäisches Patentamt
European Patent Office
Office européen des brevets

(11) EP 1 413 313 A1

(12) **EUROPEAN PATENT APPLICATION**
published in accordance with Art. 158(3) EPC

(43) Date of publication:
**28.04.2004 Bulletin 2004/18**

(21) Application number: **02743750.8**

(22) Date of filing: **27.06.2002**

(51) Int Cl.[7]: **A61K 41/00**, A61K 31/409, A61K 31/555, A61K 49/00, A61K 49/04, A61P 1/18, A61P 3/10, A61P 43/00

(86) International application number:
**PCT/JP2002/006510**

(87) International publication number:
**WO 2003/002145 (09.01.2003 Gazette 2003/02)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **27.06.2001 JP 2001194999**

(71) Applicant: **Meiji Seika Kaisha, Ltd.**
**Tokyo 104-8002 (JP)**

(72) Inventors:
- **AIZAWA, Katsuo, Tokyo Medical College Tokyo 160-0022 (JP)**
- **KANAZAWA, Masao, Tokyo Medical College Tokyo 160-0022 (JP)**
- **SAITO, Keiji, Solid Square Kawasaki-shi, Kanagawa 210-0913 (JP)**
- **IMAI, Satoshi, Solid Square Ltd. Kawasaki-shi, Kanagawa 210-0913 (JP)**

(74) Representative: **Le Coupanec, Pascale et al**
**Nony & Associés,**
**3 rue de Penthièvre**
**75008 Paris (FR)**

(54) **DIAGNOSIS AND TREATMENT OF INSULIN−DEPENDENT DIABETES MELLITUS AND INSULITIS**

(57) A photodynamic diagnosis or a photodynamic therapy of IDDM and pancreatic insulitis can be conducted by administering a fluorescent photosensitizer which has a property capable of being uptaken specifically by the macrophages having infiltrated the inflammatory sites of Langerhans islets of the pancreas induced by pancreatic insulitis, or an X-ray absorbing substance which has a property capable of being uptaken specifically by the infiltrating macrophages present in the inflammatory sites of Langerhans islets and capable of absorbing an X-ray upon irradiation of the X-ray; then either irradiating a part of the pancreas containing therein the macrophages having infiltrated the inflammatory lesion sites of pancreatic insulitis, with an excited light ray or an X-ray, and observing a fluorescence or an X-ray absorption occurring in said inflammatory sites of Langerhans islets, or irradiating the pancreas or a part thereof with a light ray or an X-ray at a high irradiance of the irradiated ray sufficient to permit that the irradiated photosensitizer or X-ray absorbing substance uptaken and accumulated in the infiltrating macrophages can damage or destroy the macrophages. There is now provided a diagnostic composition or a therapeutic composition which is suitable for the purpose of the diagnosis or therapy described above.

Figure 1

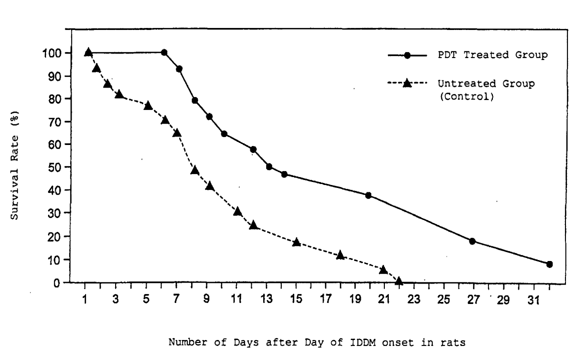


EP 1 413 313 A1

## Description

Technical Field

This invention relates to diagnostic compositions for diagnosing insulin-dependent diabetes mellitus (abbreviated sometimes as IDDM) and pancreatic insulitis by a photodynamic diagnosis (abbreviated sometimes as PDD), and to therapeutic compositions for treating therapeutically IDDM and pancreatic insulitis by a photodynamic therapy (abbreviated sometimes as PDT).

This invention also relates to a photodynamic diagnostic method and a photodynamic therapeutic method for IDDM and pancreatic insulitis, comprising administration of a photosensitizer or an X-ray absorbing substance.

Further, this invention relates to uses of a photosensitizer or an X-ray absorbing substance, in the manufacture of pharmaceutical products for diagnosing or treating IDDM and pancreatic insulitis, by a PDD method or a PDT method.

Background Art

Diabetes mellitus is a disease of which major characteristic is a chronic hyperglycemia attributable to a deficiency of the action of insulin in the body of the patient and which is accompanied by a variety of metabolic disorders characteristic to diabetes mellitus. Diabetes mellitus is classified into insulin-dependent diabetes mallitus (IDDM) and non-insulin-dependent diabetes mellitus. IDDM, which occurs clinically most in the ages of infancy or youth, is a diabetes mellitus which can be diagnosed before the 30 years of age. IDDM amounts to 10 to 15% of the whole of the patients of diabetes mellitus. In IDDM, the symptom of hyperglycemia occurs abruptly, and IDDM advances to a condition called diabetic ketoacidosis when a therapy of IDDM by administration of insulin is omitted.

Clinical onset of IDDM is said to occur due to that destruction of the insulin-secreting Langerhans islet β cells in the pancreas was induced at a high degree of 90% or more by autoimmune reactions and that the secretion of insulin from pancreas has terminated entirely or substantially.

It is reported that, even before the clinical onset of IDDM, pancreatic insulitis induced due to the autoimmune reactions has occurred already in the pancreas. Pancreatic insulitis is meant by conditions such that, due to the autoimmune reactions, the inflammation cells, including T lymphocytes, other lymphocytes and macrophages, become surrounding many pancreatic islets, namely Langerhans islets, in the pancreatic inflammatory sites, and that the lymphocytes and macrophages are present to have infiltrated the interior of the inflammatory pancreatic islets (Langerhans islets). In the inflammatory pancreatic islets which have been infiltrated by the T lymphocytes and macrophages, the insulin-secreting pancreatic islet β cells are selectively attacked or damaged, and the damaged β cells are phagocytosed by the macrophages and finally disappeared. Due to the pancreatic insulitis, the Langerhans islets present in the Langerhans islet inflammatory sites can commence to deform, and further all or a large portion of the β cells in the inflammatory sites of Langerhans islets disappeared, so that the secretion of insulin from pancreas stops and the inflammatory Langerhans islets themselves disappeared ultimately.

In order to succeed in achieving a therapy of IDDM after the onset of IDDM, it is necessary to initiate the therapy of IDDM at the earliest stage of IDDM and to supply exogenous insulin externally to a patient of IDDM who has been deprived of the ability to produce insulin in vivo. It is desirable that the therapy of IDDM is effected by conducting a therapeutic treatment to stop the autoimmune reactions causative for IDDM, so that the supply of exogenous insulin can be made unnecessary. To these ends, the progress of the pancreatic insulitis and IDDM should necessarily be stopped within a period when the patient has still retained a number of the pancreatic β cells which is enough to produce a sufficient amount of endogenous insulin.

In usual, diagnosis of IDDM is carried out mainly by judging whether patient's reactions with an antibody ICA (namely, Islet Cell Antibody) against pancreatic cells, for example an anti-insulin autoantibody (IAA) or an anti-GAD (namely, glutamic acid decarboxylase) antibody, are positive or not, and additionally by examining serum C-peptide reaction of the patient through a glucagon loading test which is a method of evaluating an ability of the patient to secrete endogenous insulin, with reference to the clinical progress of the patient such as mode of the onset of IDDM and the presence or absence of ketosis, as well as examination of HLA type [refer to Shimada's article titled Morbid State of Type I Diabetes Mellitus and its Guideline for Diagnosis: Mebio 2, pp.28-38 (2000)]. However, diagnosis of IDDM made in all of these examination criteria is not yet determinative as a reliable examination indicator for detecting the destruction of pancreatic β cells.

Further, the usual insulin therapy may be effective in treatment of IDDM, but insulin should necessarily be injected regularly every day to keep the life of the patient. Creation of a physiological pattern of spontaneous insulin secretion of patient which is attained by the supply of exogenous insulin is considered to bring about a good control of the blood-sugar level, and an enhanced insulin therapy comprising a continuous subcutaneous insulin infusion is actively used.

However, with an administered prompt insulin preparation, a time of about 2 hours is required to reach the

peak of the therapeutic action, and a time of about 6 to 8 hours passes before the therapeutic action of insulin disappears. Accordingly, there often occurs a case where, in the insulin therapy, a phenomenon such as hypoglycemia appears in 3 to 4 hours after a meal. Further, even after the insulin therapy has been initiated with IDDM patients, ketoacidosis may occur upon omission of the insulin injection or under the affection of stresses as induced by infectious diseases, accidents or severe medical treatment. Further, the insulin therapy is a symptomatic therapy and the onset of IDDM occurs in persons of young age, so that the injection of exogenous insulin is required every day for a long period of years, which gives a heavy burden on the patients. Accordingly, there is now still a demand for a new therapeutic method for IDDM, which can practically substitute for the usual insulin therapy.

As described above, IDDM onsets as a result of the selective destruction of the insulin-secreting pancreatic β cells by the autoimmune reactions, which destruction was induced to a high degree of 90% or more. Recently, it was reported that, just after the onset of IDDM in IDDM animal model, about 30% of the pancreatic β cells can remain survived without being destroyed, and also that, in the IDDM animal model, just before the onset of IDDM, the total volume of the pancreatic β cells can maintain their original volume [refer to Shimada et al's, articles; Diabetes, 45, pp. 1063-1067 (1996)]. Further, it is also reported for humans that human patients of anti-GAD-antibody-positive diabetics who have an ameliorated ketosis by a dietary cure, or human patients of autoantibody-positive diabetics who have developed diabetes mellitus by ketoacidosis, can become needless to be supplemented with exogenous insulin because the disappearance of autoantibody was attained [refer to Morimoto et al, Diabetes care, 21 (11), pp.2037-2039 (1998)].

From these reports of Shimada et al and Morimoto et al's articles, it can be estimated that a large number of the surviving pancreatic β cells remain alive still at an initial stage immediately before or after the onset of IDDM in humans without being destroyed by the autoimmune reactions. Accordingly, if early diagnosis of IDDM can be made possible and if a therapeutic treatment for inhibition or termination of the autoimmune reactions causative for IDDM, can be conducted at an early stage immediately before or after the onset of IDDM, namely if early therapy of IDDM can be effected thereby, the destruction of pancreatic β cells can be minimized, while the Langerhans islets can still secure their ability to produce endogenous insulin. When early treatment of IDDM can thus be accomplished, the IDDM patient can be set free from the long term injection of exogenous insulin.

For study of IDDM, there are fortunately available many kinds of useful animal models such as BB rats and NOD mice, and such IDDM animal models can be used to study the mechanism of the onset of IDDM as well as therapeutic methods for IDDM (refer to Rossini et al, Ann Rev. Immunol., 3, pp. 289-320 (1985)).

It is known that in particular, BB rats, either male or female, can develop IDDM on Day 60 to Day 120 after birth, and IDDM as developed in the BB rats is similar in many respects to human IDDM (Like AA et al's article, J. Exp. Med., 164, pp. 1145-1159 (1986). Thus, it is known that the BB rats develop typical IDDM due to a known cause that, based on their hereditary background, pancreatic insulitis, namely the inflammation in the Langerhans islets occurs via the mechanism of the autoimmune reactions, which lead to the destruction of the pancreatic β cells present in the inflammatory sites in the Langerhans islets. It is found that the inflammation immune cells having infiltrated the inflammatory sites of the Langerhans islets having the pancreatic insulitis upon an onset of IDDM in BB rats are T lymphocytes, NK cells, macrophages, neutrophils, eosinophils and others. It is thought that at first, macrophages begin to infiltrate the inflammatory sites of Langerhans islets and then T lymphocytes, NK cells and macrophages infiltrate the inflammatory sites so that these inflammation cells such as T lymphocytes and macrophages can participate in the destruction and disappearance of the β cells in the Langerhans islets (refer to Like AA et al., Springer-Verlag, The pathology of the Endocrine Pancreas in Diabetes, pp.269-284 (1988) and Like AA et al's article ., J. Exp. Med. 164, pp.1145 (1986)). It is also known that, in human IDDM, the infiltration of the inflammation cells such as T lymphocytes and macrophages into the inflammatory sites of Langerhans islets occur in the pancreatic insulitis of a human.

It is known that the T lymphocytes and NK cells having infiltrated the Langerhans islets can attack and damage pancreatic β cells and the damaged pancreatic β cells can be eaten by the infiltrating macrophages to disappear. Consequently, the pancreatic β cells present in the inflammatory sites of Langerhans islets are destroyed by the various inflammation cells (the immune cells) having infiltrated the inflammatory sites of Langerhans islets.

Under the technical background as mentioned above, we, the present inventors, have now attained a concept that, when the macrophages having infiltrated the inflammatory sites of Langerhans islets can selectively be damaged or destroyed at an early stage immediately before or immediately after the onset of IDDM, permitting that the pancreatic β cells can be prevented from being phagocytosed and destroyed by the infiltrating macrophages, thereby to protect the pancreatic β cells to a certain degree, it would then be possible that the pancreatic insulitis caused by the autoimmune reactions in human can be delayed from occurring and also the onset of human IDDM can be controlled or delayed, and in this respect, a therapeutic treatment of human IDDM can be effected.

Disclosure of the Invention

An object of this invention is to provide some measures capable of making an early diagnosis of pancreatic

insulitis caused by the autoimmune reactions and early diagnosis of IDDM, as well as some measures capable of making an early therapeutic treatment of pancreatic insulitis and IDDM. Particular objects of this invention are to provide a diagnostic composition which can be used effectively in a photodynamic diagnosis (PDD) of pancreatic insulitis and IDDM, as well as a therapeutic pharmaceutical composition which is effective in a photodynamic therapy (PDT) of pancreatic insulitis and IDDM.

Further objects of this invention are to provide a method which can make an early diagnosis of pancreatic insulitis and IDDM by PDD, as well as a method which can make an early therapy of pancreatic insulitis and IDDM by PDT.

We, the present inventors, have conducted investigations in order to achieve the objects of this invention described above, and as a result, the present inventors have now found for the first time that a certain kind of photosensitizer upon its administration has a combination of a property capable of being specifically uptaken by the macrophages having infiltrated the inflammatory sites of Langerhans islets in the pancreas of a mammal with IDDM but not capable of being uptaken by the pancreatic α cells and β cells; and a property capable of being activated upon absorption of a light ray of a specific wavelength to emit a fluorescence. Further, the present inventors have now found that, when a photosensitizer having the aforesaid properties in combination was administered and then the inflammatory sites of Langerhans islets containing therein the macrophages, where the photosensitizer having the aforesaid properties has been uptaken and accumulated, are irradiated with a selected light ray of a specific wavelength, detection of the inflammation lesion sites of pancreatic insulitis as well as a photodynamic diagnosis and a therapeutic treatment of the pancreatic insulitis and IDDM are possible to be conducted by using the visible florescence developed. Thus, it has now been found that, when there is conducted a method which comprises administering to a mammal with IDDM either a pharmaceutical composition containing a photosensitizer having the aforesaid properties as an active ingredient, or said photosensitizer alone; then allowing time to elapse for a period of time sufficient to permit the administered photosensitizer having the aforesaid properties to be uptaken specifically by the macrophages having infiltrated the inflammatory sites of Langerhans islets in the pancreas of the mammal with IDDM; and after the elapse of such sufficient time period, irradiating the inflammatory sites of Langerhans islets where the infiltrating macrophages having uptaken the administered photosensitizer therein are present, with a selected light ray of a specific wavelength suitable for photo-excitation of the administered photosensitizer, thereby enabling the photosensitizer as uptaken in said macrophages to emit the visible fluorescence unique to said photosensitizer, it is feasible to observe a fluorescence image of the infiltrating macrophages, and to detect the inflammatory sites of Langerhans islets containing the macrophages with the fluorescence image, with reference to the emitting fluorescence by an endoscopic or laparoscopic observation. Therefore, it has now been found at this time that the onset of pancreatic insulitis and the onset of IDDM can be diagnosed through a photodynamic method by detecting or observing the fluorescence-emitting inflammatory sites of Langerhans islets containing the macrophages having shown the fluorescence image, by means of endoscope or laparoscope.

The present inventors have now recognized that a known compound used as a photosensitizer in conventional methods of PDT, which is mono-L-aspartyl chlorin e6 represented by the following formula (I):

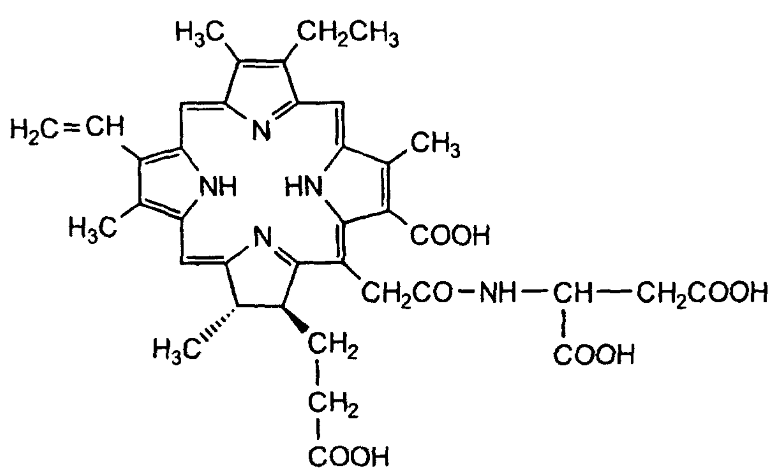

(I)

or a pharmaceutically acceptable salt or a solvate thereof, such as a hydrate or ethanol-adduct thereof, is very excellent and effective for use in the above-mentioned PDD method. Mono-L-aspartyl chlorin e6 has a general name "talaporfin" (see WHO Drug Information, Vol.15, No.1 (2001), Recommended INN: List 45).

Further, the present inventors have now found that, when various photosensitizers utilized generally as photosensitive agents in a conventional method of PDT, for example, known chlorin e6, chlorin e6 derivatives, benzoporphyrin derivatives, etiopurpurin, Lu-Tex, ATX-S10, Foscan (trade name) or Photofrin (trade name) (refer to e.g., JP patent publication Hei-6-88902, JP patent Publication Hei-6-89000, and US Patent Nos. 4, 675, 338; 6,162,242; 5, 095, 030 and 5, 756, 541) are each singly administered, these photosensitizers have a property such that they can specifically be uptaken by and into the macrophages having infiltrated the inflammatory sites of Langerhans islets but cannot be uptaken by the pancreatic cells in the pancreas of a mammal with IDDM.

Incidentally, the specification of US Patent No. 4,693,885 of Bommer et al., discloses various kinds of photosensitive tetrapyrrole derivatives such as chrolin e6 amide derivatives. It further discloses a method for detection of a tumor, which comprises administrating a photosensitive chlorin e6 derivative such as mono-L-aspartyl chlorin e6 sodium salt to a mammal, then irradiating a part to be examined of said mammal with a light ray of a sufficient wavelength, and thereby observing a fluorescence emitted from the tumor-affected part, as well as a method for diagnosis of a tumor, which comprises administrating a photosensitive chlorin e6 derivative to a mammal bearing a tumor, and then irradiating the mammal with a light ray having an intensity and a wavelength sufficient to activate the said chlorin derivative, thereby to exert the cell-killing effect against the tumor.

Further, the present inventors have now found from our recent studies that, when there is administered an X-ray absorbing substance which has a property such that, upon irradiation with an X-ray of a wavelength of 0.153Å, said substance can absorb the X-ray of such wavelength, and which substance is talaporfin-gold complex represented by the following formula (II):

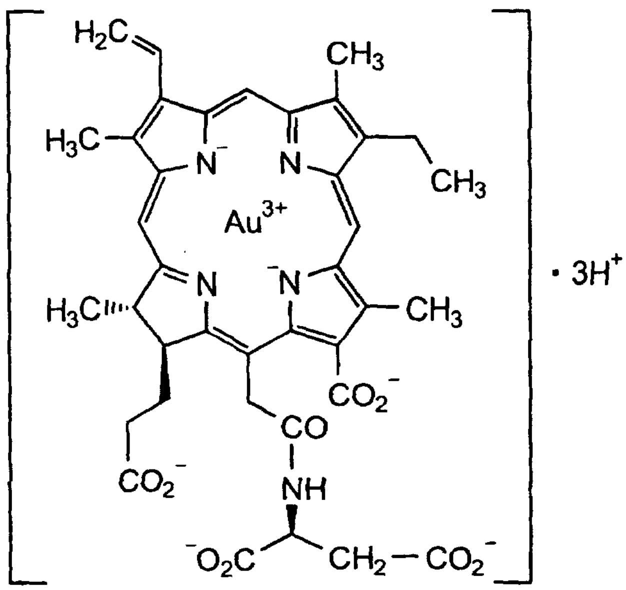

(II)

or a pharmaceutically acceptable salt thereof or a solvate thereof, e.g. a hydrate thereof (refer to PCT Application International Publication No. WO 00/02882) , said gold complex can exhibit a property such that the gold complex can be uptaken specifically by the macrophages having infiltrated the inflammatory sites of Langerhans islets but cannot be uptaken by the Langerhans islet cells in the pancreas of a mammal with IDDM.

We, the present inventors, have now found that, in particular, when the talaporfin-gold complex of the above formula (II) is administered to a mammal with IDDM, the gold complex of formula (II) administered can be uptaken specifically by the macrophages having infiltrated the inflammatory sites of Langerhans islets in the pancreas to accumulate in the macrophages which are present in the inflammatory sites of Langerhans islets. When an X-ray of a wavelength of 0.153 Å is applied at a sufficient irradiance from an X-ray generator placed in the outside of the body of the mammal selectively to the pancreas, the gold complex of formula (II) , which has accumulated in the macrophages having infiltrated the inflammatory sites of Langerhans islets in the pancreas and also having contained the uptaken gold complex of formula (II), is able to absorb the X-ray, and thus an image of the X-ray absorption is produced in the inflammatory sites of Langerhans islets and can be observed and detected by means of X-ray radiography, with photographying the X-ray absorption image on an X-ray film placed outside the body of the mammal. Therefore, by conducting a method comprising administrating the compound of formula (II) to a mammal and photographying the pancreas selectively by an X-ray radiography, it is feasible to observe and detect the X-ray absorption image produced in the pancreas and hence to detect the pancreatic insulitis and diagnose the inflammatory sites of Langerhants islets in

the pancreas.

On the basis of the inventors' finding described above, this invention has been accomplished.

Accordingly, in a first aspect of this invention, there is provided a diagnostic composition for diagnosing insulin-dependent diabetes mellitus and pancreatic insulitis accompanied by destruction of pancreatic β cell, which comprises as an active ingredient a photosensitizer having a property capable of being uptaken specifically by the macrophages having infiltrated the inflammatory sites of Langerhans islets in the pancreas of a mammal with the insulin-dependent diabetes mellitus, as well as a property capable of being activated upon absorption of a light ray of a specific wavelength to emit a visible fluorescence, in combination with a pharmaceutically acceptable carrier for the active ingredient.

In the diagnostic composition according to the first aspect of the invention, the photosensitizer to be incorporated therein may be chlorin e6, a chlorin e6 derivative, a benzoporphyrin derivative, etiopurpurin, Lu-Tex, ATX-S10, Foscan (trade name) and Photofrin (trade name), or a pharmaceutically acceptable salt thereof.

The photosensitizer to be given as an effective agent in the diagnostic composition according to the first aspect of the invention may preferably be talaporfin represented by formula (I):

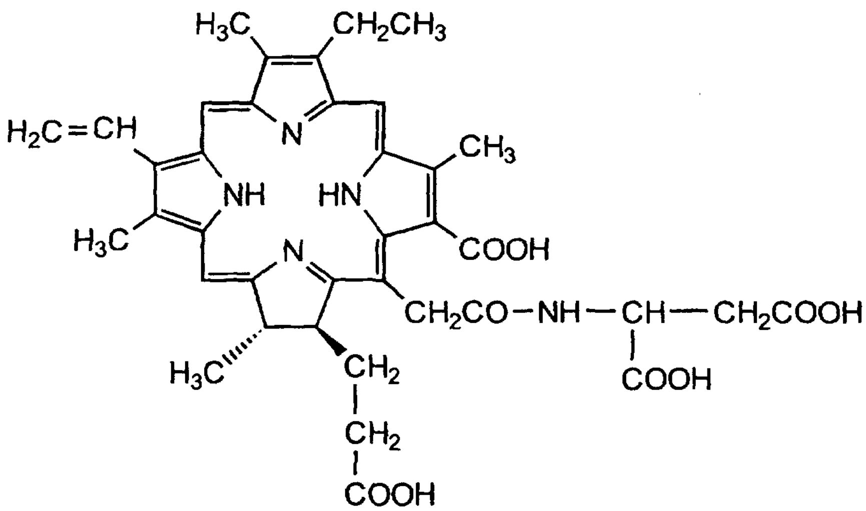

(I)

or a pharmaceutically acceptable salt or a solvate thereof, for example, a hydrate or an ethanol-adduct thereof.

In the diagnostic composition of the first aspect of this invention, the carrier incorporated therein may be a pharmaceutically acceptable solid or liquid carrier such as starch, crystalline cellulose, glucose, water, physiological saline, aqueous solution of glucose, ethanol and aqueous ethanol. This diagnostic composition may be prepared in the form of orally administrable tablets or capsules and also may be prepared in the form of intravenously injectable, subcutaneously injectable or intramuscularly injectable and sterile solution or aqueous dispersion. This composition may also be prepared in the form of a lyophilized preparation for injection. The proportion of the photosensitizer present as the active ingredient in the diagnostic composition may be in a range of 1% to 5% by weight of the composition.

The diagnostic composition of the first aspect invention may preferably be administered, for example, intravenously, intramuscularly or subcutaneously or via a pancreatic duct to a mammal to be examined.

The diagnostic composition of the first aspect invention can be used and administered in a method for detection of IDDM and pancreatic insulitis according to a second aspect of this invention, as well as in a method for diagnosis of IDDM and pancreatic insulitis according to a third aspect of this invention as described hereinafter.

In a second aspect of this invention, there is provided a method for detecting insulin-dependent diabetes mellitus and pancreatic insulitis, which comprises:

administering to a mammal to be diagnosed an effective amount of a photosensitizer or a pharmaceutical composition containing said photosensitizer as an active ingredient, said photosensitizer having a property capable of being uptaken specifically by the macrophages having infiltrated the inflammatory site of Langerhans islets in the pancreas of the mammal, as well as a property capable of being activated upon absorption of a light ray of a specific wavelength to emit a visible fluorescence;

irradiating at least a part of the pancreas with a light ray of a specific wavelength suitable for photo-excitation of the administered photosensitizer, at a sufficient irradiance of the irradiated light ray, whereby the visible fluorescence is caused to emit in the pancreas from the photosensitizer present in the macrophages having uptaken said photosensitizer therein;

observing the visible fluorescence emitted in the inflammatory sites; and
detecting by such observation of the fluorescence the inflammatory sites of Langerhans islets occurring in the pancreas.

In a third aspect of this invention, there is provided a method for diagnosing insulin-dependent diabetes mellitus and pancreatic insulitis accompanied by destruction of pancreatic islet β cells, by the photodynamic diagnosis method, characterized in that the method comprises:

administering to a mammal to be diagnosed an effective amount of a photosensitizer or a pharmaceutical composition containing said photosensitizer as an active ingredient, said photosensitizer having a property capable of being uptaken specifically by the macrophages having infiltrated the inflammatory sites of Langerhans islets in the pancreas of the mammal with the insulin-dependent diabetes mellitus, as well as a property capable of being activated upon absorption of a light ray of a specific wavelength to emit a visible fluorescence ;
allowing time to elapse for a sufficient time which permits the administered photosensitizer to have been uptaken in the macrophages having infiltrated the inflammatory sites of Langerhans islets in the pancreas of the mammal;
and after the elapse of such sufficient time, irradiating at least a part of the pancreas or the inflammatory sites of Langerhans islets where the infiltrating macrophages having uptaken the administered photosensitizer therein are present, with a selected light ray of a specific wavelength suitable for photo-excitation of the administered photosensitizer, at a sufficient irradiance of the irradiated light ray, whereby the visible fluorescence is caused to emit from said photosensitizer contained in the macrophages present in the inflammatory sites of Langerhans islets as infiltrated by said macrophages containing said photosenitizer therein, so that the macrophages present in the inflammatory sites of Langerhans islets can selectively show a fluorescence image;
observing by an endoscope the presence or absence of the inflammatory sites containing the macrophages which show the fluorescence image;
detecting by such observation the presence of the inflammatory sites of Langerhans islets which show the fluorescence image; and
diagnosing by such detection an onset of pancreatic insulitis accompanied by destruction of pancreatic islet β cells, and an onset of insulin-dependent diabetes mellitus caused by the pancreatic insulitis.

In the detection method of the second aspect invention and in the diagnosis method of the third aspect invention as described above, the administered photosensitizer may be talaporfin of the formula (I) shown hereinbefore or a pharmaceutically acceptable salt thereof, particularly talaporfin tetrasodium salt. When the administered photosensitizer is talaporfin tetrasodium salt, this may preferably be administered by intravenous injection at a dose of 0.1 mg/kg to 5 mg/kg.

In the methods of the second aspect invention and of the third aspect invention, the administered photosensitizer may be talaporfin tetrasodium salt, and after intravenous injection of talaporfin tetrasodium salt, at least a part of the pancreas or the inflammatory sites of Langerhans islets may preferably be irradiated with a red-colored laser light containing a light of 664 nm wavelength by means of a laparoscope or by means of an endoscope inserted into the pancreatic duct, or by means of an endoscope inserted in the gastric cavity or duodenal cavity. Further, the visible fluorescence emitted selectively from the macrophages present in the inflammatory sites of Langerhans islets can be detected and observed by means of a laparoscope placed near to said inflammatory sites or by means of an endoscope inserted in the pancreatic duct.

In a fourth aspect of this invention, there is provided use of talaporfin or a pharmaceutically acceptable salt thereof, as a photosensitizer, in the manufacture of a pharmaceutical product for diagnosing by the photodynamic diagnosis method the insulin-independent diabetes mellitus and pancreatic insulitis accompanied by destruction of pancreatic β cells.

In a fifth aspect of this invention, there is provided a diagnostic composition for diagnosing insulin-dependent diabetes mellitus and pancreatic β cells, characterized in that the composition comprises as an active ingredient an X-ray absorbing substance having a property capable of being uptaken specifically by the macrophages having infiltrated the inflammatory sites of Langerhans islets in the pancreas of a mammal with the insulin-dependent diabetes mellitus, as well as a property capable of absorbing an X-ray upon receiving irradiation of the X-ray, in combination with a pharmaceutically acceptable carrier for the active ingredient.

In the diagnostic composition of the fifth aspect invention, the X-ray absorbing substance may be a talaporfin-gold complex having the following for (II):

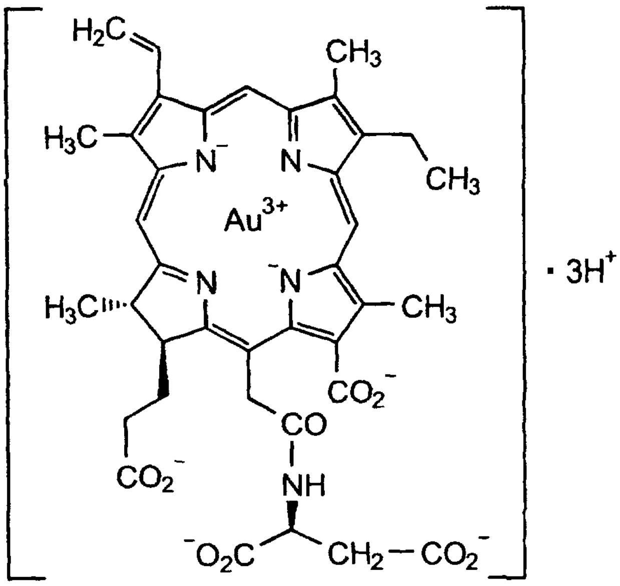

(II)

or a pharmaceutically acceptable salt or a solvate thereof, e.g. a hydrate or an ethanol-adduct thereof. The diagnostic composition containing the aforesaid X-ray absorbing substance according to the fifth aspect invention may preferably be administered intravenously, intramuscularly or subcutaneously or via a pancreatic duct to the mammal to be examined.

In the diagnostic composition of the fifth aspect invention, the carrier to be mixed with the X-ray absorbing substance as an active ingredient may be same as the carrier which is incorporated in the diagnostic composition of the first aspect invention. This diagnostic composition may be formulated in the same manner as the diagnostic composition of first aspect invention is formulated.

The diagnostic composition of the fifth aspect invention may be used in a method for detection of IDDM and pancreatic insulitis according to a sixth aspect of this invention, as well as in a method for diagnosis of IDDM and pancreatic insulitis according to a seventh aspect of this invention as described hereinater.

In a sixth aspect of this invention, there is provided a method for detecting insulin-dependent diabetes mellitus and pancreatic insulitis, which comprises:

administering to a mammal to be examined an effective amount of an X-ray absorbing substance having a property capable of being uptaken specifically by the macrophages having infiltrated the inflammatory sites of Langerhans islets in the pancreas of a mammal with the insulin-dependent diabetes mellitus, as well as a property capable of absorbing an X-ray upon receiving irradiation of the X-ray;
then irradiating selectively the pancreas of the mammal with an X-ray beam containing an X-ray of a specific wavelength as delivered from an X-ray generator placed outside the body of the mammal to be examined;
observing the X-ray absorption occurring in the pancreas, by means of an X-ray radiography; and
detecting by such X-ray radiographic observation the inflammatory sites of Langerhans islets.

In a seventh aspect of this invention, there is provided a method for diagnosing insulin-dependent diabetes mellitus and pancreatic insulitis accompanied by destruction of pancreatic β cells, characterized in that the method comprises:

administering to a mammal to be diagnosed an effective amount of an X-ray absorbing substance or a pharmaceutical composition containing said X-ray absorbing substance as an active ingredient, said X-ray absorbing substance having a property capable of being uptaken specifically by the macrophage having infiltrated the inflammatory sites of Langerhans islets in the pancreas of a mammal with the insulin-dependent diabetes mellitus, as well as a property capable of absorbing an X-ray upon receiving irradiation of the X-ray;
allowing time to elapse for a sufficient time which permits the administered X-ray absorbing substance to have been uptaken in the macrophages having infiltrated the inflammatory sites of Langerhans islets in the pancreas of the mammal;
and after the elapse of such sufficient time; irradiating selectively the pancreas of the mammal with an X-ray beam containing an X-ray of a specific wavelength as delivered from an X-ray generator placed outside the body of the

mammal to be examined, at a sufficient irradiance of the irradiated X-ray;
receiving on an X-ray film the X-ray beam having passed through the body of the mammal to take a photography of the X-ray absorption occurring in the pancreas;
observing in the photographed X-ray absorption image of said X-ray film the presence or absence of the X-ray absorption image shown by the group of the macrophages having infiltrated the inflammatory sites of Langerhans islets and having uptaken specifically therein the administered X-ray absorbing substance;
detecting by such X-ray radiographic observation the presence of the inflammatory sites of Langerhans islets, with reference to the photographed X-ray absorption image of the X-ray film; and
diagnosing by such detection of the inflammatory sites of Langerhans islets an onset of pancreatic insulitis accompanied by destruction of pancreatic β cells, and an onset of insulin-dependent diabetes mellitus caused by the pancreatic insulitis.

In the detection method of the sixth aspect invention and in the diagnosis method of the seventh aspect invention as described above, the administered X-ray absorbing substance may be a talaporfin-gold complex of the formula (II) shown hereinbefore or a pharmaceutically acceptable salt thereof, particularly tetrasodium salt thereof. When the administered X-ray absorbing substance is a tetrasodium salt of the talaporfin-gold complex, this may preferably be administered by intravenous injection at dose of 0.1 mg/kg to 5 mg/kg.

When the method of the sixth aspect invention or the method of the seventh aspect invention is carried out with administration of talaporfin-gold complex tetrasodium salt as the X-ray absorbing substance, it is preferable that, after intravenous injection of the talaporfin-gold complex tetrasodium salt, the pancreas is irradiate with an X-ray beam containing an X-ray of 0.153 Å wavelength delivered externally from the outside of the body of the mammal.

In an eighth aspect of this invention, there is provided use of a talaporfin-gold complex or a pharmaceutically acceptable salt thereof, as an X-ray absorbing substance, in the manufacture of a pharmaceutical product for diagnosing by the photodynamic diagnosis method the insulin-dependent diabetes mellitus and pancreatic insulitis accompanied by destruction of pancreatic β cells.

When the photosensitizer used as an active ingredient in the composition of the first aspect invention, for example, the compound of formula (I) above, is administered into a human or a mammal with IDDM, the administered photosensitizer is uptaken selectively and specifically by the macrophages having infiltrated and present in the inflammatory sites of Langerhans islets, that is, the pancreatic lesion sites of IDDM. The photosensitizer uptaken in the infiltrating macrophages, upon being irradiated with a light ray having a sufficient irradiance and a wavelength suitable for photo-excitation of the photosensitizer, can be activated by absorption of the light ray and emit a visible fluorescence. When the intensity of irradiation of the light ray is then increased, the photosensitizer uptaken and accumulated in the macrophages is activated by absorption of the light ray, to generate active oxygen and thereby exhibit a cell-killing effect on the macrophages, whereby the macrophages can be damaged or destroyed. In particular, the compound of formula (I), upon the irradiation with a red laser light containing a light of a wavelength of 664 nm, can be activated by absorption of the 664 nm light ray, to generate active oxygen and thereby exhibit a cell-killing effect on the macrophage.

When the X-ray absorbing substance, for example, the compound of formula (II) , which is used as an active ingredient of the diagnostic composition of the fifth aspect invention, is irradiated with an X-ray beam containing an X-ray of a specific wavelength, for example a wavelength of 0.153Å, it is activated by absorption of the X-ray, and it can exhibit an electron-releasing function by irradiation of the X-ray to exert the cell-killing effect. Upon irradiation of the X-ray beam, therefore, the macrophages containing the X-ray absorbing substance therein can be damaged or destroyed.

Accordingly, when the photosensitizer used as the active ingredient in the first aspect invention as well as the X-ray absorbing substance used as the active ingredient in the fifth aspect invention, have been uptaken in the macrophages and are then irradiated with a light ray or an X-ray at an irradiance sufficient to exert their cell-killing effect, they can damage or destroy the macrophages having infiltrated the inflammatory sites of Langerhans islets, whereby the inflammatory sites of Langerhans islet can be treated therapeutically, to achieve a photodynamic therapy (PDT) of the pancreatic insulitis and IDDM caused by pancreatic insulitis.

Accordingly in an eighth aspect of the invention, there is provided a therapeutic composition for treating therapeutically insulin-dependent diabetes mellitus and pancreatic insulitis accompanied by destruction of pancreatic β cells, which comprises as an active ingredient a photosensitizer having a property capable of being uptaken specifically by the macrophages having infiltrated the inflammatory sites of Langerhans islets in the pancreas of a mammal with the insulin-dependent diabetes mellitus, as well as a property capable of being activated upon absorption of a light ray of a specific wavelength to emit a visible fluorescence, in combination with a pharmaceutically acceptable carrier for the active ingredient.

The photosensitizer used as an active ingredient in the therapeutic composition of the eighth aspect of the invention may be chlorin e6, a chlorin e6 derivative, a benzoporphyrin derivative, etiopurpurin, Lu-Tex, ATX-S10, Foscan and Photofrin, or a pharmaceutically acceptable salt thereof.

The photosensitizer to be administered as an active ingredient in the therapeutic composition of the eighth aspect of the invention may preferably be talaporfin of formula (I) above, or a pharmaceutically acceptable salt thereof or a solvate thereof, and the light ray to be irradiated in this case may be red laser lights containing a light of a wavelength of 664 nm.

In the therapeutic composition of the eighth aspect invention, the carrier to be mixed therein may be the same as the carrier used in the composition of the first aspect invention, and the proportion of the active ingredient in the composition may be the same as in the composition of the first aspect invention.

The therapeutic composition of the eighth aspect invention may be administered, for example, intravenously, intramuscularly or subcutaneously or via a pancreatic duct to a patient with IDDM, and the composition of the eighth aspect invention may be used in a photodynamic method for treating IDDM and pancreatic insulitis as described hereinafter.

In a ninth aspect of this invention, there is provided a method for treating therapeutically insulin-dependent diabetes mellitus and pancreatic insulitis accompanied by destruction of pancreatic β cells, by the photodynamic therapy method, characterized in that the method comprises:

administering to a mammal to be treated an effective amount of a photosensitizer or a pharmaceutical composition containing said photosensitizer as an active ingredient, said photosensitizer having a property capable of being uptaken specifically by the macrophages having infiltrated the inflammatory sites of Langerhans islets in the pancreas of the mammal with the insulin-dependent diabetes mellitus, as well as a property capable of being activated upon absorption of a light ray of a specific wavelength to emit a visible fluorescence;
allowing time to elapse for a sufficient time which permits the administered photosensitizer to have been uptaken in the macrophages having infiltrated the inflammatory sites of Langerhans islets in the pancreas of the mammal;
and after the elapse of such sufficient time, irradiating at least a part of the pancreas or the inflammatory sites of Langerhans islets where the infiltrating macrophages having uptaken the administered photosensitizer therein are present, with a selected light ray of a specific wavelength suitable for photo-excitation of the administered photosensitizer, with the irradiation of the selected light ray being effected at an irradiance of the irradiated light ray sufficient to permit that the photosensitizer uptaken and accumulated in the macrophages having infiltrated the inflammatory sites of Langerhans islets is activated by the absorption of the irradiated light ray to generate active oxygen and to damage or destroy said macrophages by active oxygen;
damaging or destroying by such light irradiation the macrophages which infiltrate the inflammatory sites of Langerhans islets; and
inhibiting by such damage or destroy of macrophages the destruction of the pancreatic β cells caused by initial infiltration of the macrophages and subsequent infiltration of the various inflammatory cells, whereby to suppress or treat therapeutically the pancreatic insulitis and the insulin-dependent diabetes mellitus caused by the pancreatic insulitis.

In the therapeutic method of the ninth aspect invention, the photosensitizer to be administered may be talaporfin or a pharmaceutically acceptable salt thereof, particularly talaporfin tetrasodium salt. When the administered photosensitizer is talaporfin tetrasodium salt, this may preferably be administered by intravenous injection at a dose of 0.1 mg/kg to 5 mg/kg. In case the administered photosensitizer is talaporfin tetrasodium salt, it is preferable that, after intravenous injection of talaporfin tetrasodium salt, at least a part of the pancreas or the inflammatory sites of Langerhans islets is or are irradiated with a red-colored laser light containing a light of 664 nm wavelength by means of a laparoscope or by means of an endoscope inserted in the gastric cavity or duodenal cavity, at a light irradiance of 20 to 200 J/cm$^2$.

In a tenth aspect of this invention, there is provided use of talaporfin or a pharmaceutically acceptable salt thereof, as a photosensitizer, in the manufacture of a pharmaceutical product for treating by the photodynamic therapy method the insulin-dependent diabetes mellitus and pancreatic insulitis accompanied by destruction of pancreatic β cells.

In an eleventh aspect of this invention, there is provided a therapeutic composition for treating therapeutically insulin-dependent diabetes mellitus and pancreatic insulitis accompanied by destruction of pancreatic β cells, characterized in that the composition comprises as an active ingredient an X-ray absorbing substance having a property capable of being uptaken specifically by the macrophages having infiltrated the inflammatory sites of Langerhans islets in the pancreas of a mammal with the insulin-dependent diabetes mellitus, as well as a property capable of absorbing an X-ray upon receiving irradiation of the X-ray and capable of being activated by such X-ray absorption to display the electron-releasing function that can damage or destroy the macrophages, in combination with a pharmaceutically acceptable carrier for the active ingredient.

In the composition of the eleventh aspect invention, the X-ray absorbing substance may be a talaporfin-gold complex of formula (II) shown hereinbefore, or a pharmaceutically acceptable salt or a solvate thereof.

In a twelfth aspect of this invention, there is provided a method for treating therapeutically insulin-dependent diabetes mellitus and pancreatic insulitis accompanied by destruction of pancreatic β cells, by the photodynamic therapy method in that the method comprises:

administering to a mammal to be treated an effective amount of an X-ray absorbing substance or a pharmaceutical composition containing said X-ray absorbing substance as an active ingredient, said X-ray absorbing substance having a property capable of being uptaken specifically by the macrophages having infiltrated the inflammatory sites of Langerhans islets in the pancreas of a mammal with the insulin-dependent diabetes mellitus, as well as a property capable of absorbing an X-ray upon receiving irradiation of the X-ray and capable of being activated by such X-ray absorption to display the electron-releasing function that can damage or destroy the macrophages;
allowing time to elapse for a sufficient time which permits the administered X-ray absorbing substance to have been uptaken in the macrophages having infiltrated the inflammatory sites of Langerhans islets in the pancreas of the mammal;
and after the elapse of such sufficient time, irradiating selectively the pancreas of the mammal with an X-ray beam containing an X-ray of a specific wavelength delivered from an X-ray generator placed outside the body of the mammal to be treated, at a sufficient irradiance of the irradiated X-ray, with the irradiation of the X-ray being effected at an adjusted irradiance of the irradiated X-ray sufficient to permit that the X-ray absorbing substance uptaken and accumulated in the macrophages having infiltrated the inflammatory sites of Langerhans islets is activated by the absorption of the irradiated X-ray to display the electron-releasing function that can damage or destroy the macrophages;
damaging or destroying by such X-ray irradiation the macrophages which infiltrate the inflammatory sites of Langerhans islets; and
inhibiting by such damage or destroy of the macrophages the destruction of the pancreatic β cells caused by initial infiltration of the macrophages and subsequent infiltration of the various inflammatory cells, whereby to suppress or treat therapeutically the pancreatic insulitis and the insulin-dependent diabetes mellitus caused by the pancreatic insulitis.

In the therapeutic method of the twelfth aspect invention, the administered X-ray absorbing substance may preferably be a talaporfin-gold complex of the formula (II) or a pharmaceutically acceptable salt thereof, particularly tetrasodium salt thereof. When the administered X-ray aborbing substance is a tetrasodium salt of the talaporfin-gold complex, this may preferably be adminisitered by intravenous injection at a dose of 0.1 mg/kg to 5 mg/kg.
In the therapeutic method of the twelfth aspect invention wherein the administered X-ray absorbing substance is talaporfin-gold complex tetrasodium salt, it is possible that, after intravenous injection of the talaporfin-gold complex tetrasodium salt, the pancreas is irradiated with an X-ray beam containing an X-ray of 0.153 Å wavelength delivered externally from outside of the body of the mammal, at an energy of 5 keV to 50 keV of the irradiated X-ray.
A further aspect of this invention includes use of a talaporfin-gold complex or a pharmaceutically acceptable salt thereof as an X-ray absorbing substance, in the manufacture of a pharmaceutical product for treating therapeutically by the photodynamic therapy method the insulin-dependent diabetes mellitus and pancreatic insulitis accompanied by destruction of pancreatic β cells.

## Best Mode for Carrying Out the Invention

In the diagnostic compositions according to this invention and also in the therapeutic compositions according to this invention, the compound to be used as the active ingredient may be mixed with a pharmaceutically acceptable carrier. For example, these compositions of this invention can be in the form of a solution of the active ingredient in a suitable liquid carrier, for example, physiological saline, a glucose solution in water, and others. The composition of the invention in this solution form can be administered by a suitable method to the body of a patient to be diagnosed or treated. The composition can be formulated not only into an aqueous solution but also into an aqueous suspension having the active ingredient dispersed in water with aid of a suitable dispersant. Administration of the composition may be conducted preferably by direct injection into a blood vessel, but may be conducted also by muscular or subcutaneous administration. Further, topical administration via a pancreatic duct can be conducted with using a catheter inserted in the pancreatic duct.
The compositions according to the invention can contain at least one of a known binder, a pH adjusting agent such as phosphoric acid, hydrochloric acid or sodium hydroxide, an excipient, a preservative such as para-hydroxybenzoic acid derivative, a solvent such as water, ethanol or glycol, and an isotonizing agent such as sugar or sodium chloride. These compositions according to the invention can contain the active ingredient substance, for example, the compound of formula (I) or the compound of formula (II) in the form of its salt with a base, for example, a sodium salt, and can be formulated into a sterile, pyrogen-free lyophilized preparation.

The photosensitizer or the X-ray absorbing substance to be used in the respective aspects of the invention is preferred to have the following characteristics:

(a) When it is not activated with irradiation of light ray or X-ray or until it is activated with irradiation of light ray or X-ray, it shall be nontoxic to the mammal to whom it is given at a dose necessary for the diagnosis and therapy.
(b) It can be uptaken specifically by the macrophage having infiltrated the inflammatory lesion sites of Langerhans islets present in the pancreas with IDDM but it cannot be uptaken by the Langerhans islets cells.
(c) It can be selectively activated upon irradiation of a light ray of a specific wavelength, for example, a light ray such as visible light, electromagnetic waves, X-rays and others.
(d) Upon being irradiated with a light ray of a specific wavelength, it can emit a specific, measurable and visible fluorescence or it can absorb the irradiated X-ray.
(e) Upon irradiation of a light ray or an X-rays of a specific wavelength, it can be activated to damage or kill selectively the macrophages having infiltrated the inflammatory lesion sites of Langerhans islets, without affecting the normal tissue regions of the body which surround the Langerhans islets.
(f) After IDDM diagnosis or therapy was done, it can be easily metabolized in the body or can be excreted out of the body of human or animal.

As the photosensitizer or the X-ray absorbing substance having all the characteristics described above, talaporfin of formula (I) or its salt and the talaporfin-gold complex of formula (II) or its salt given hereinafter can be mentioned.

The sodium salt of talaporfin of formula (I) and the sodium salt of the talaporfin-gold complex of formula (II), which are used preferably according to the invention, have the characteristics (a) to (f) described above, and such sodium salts are advantageous in that they can be dissolved at a suitable solubility in a water having a physiological hydrogen ion concentration (pH). Further, it has now been found that the compound of formula (I) can emit a visible fluorescence of higher intensity than by the same dosage of other photosensitizers. Accordingly, when the sodium salt of talaporfin or talaporfin-gold complex is administered in this invention or used as the active ingredient in this invention, such sodium salt can be distributed stably and uniformly at a higher concentration in the inflammatory site of Langerhans islets containing pancreatic β cells than in the normal tissues surrounding the inflammatory lesion sites of Langerhans islets, and hence it is possible to afford a fluorescence image or an X-ray absorption image having more significant contrast.

In particular, talaporfin of formula (I) or its salt can be excited by irradiation of red laser lights containing a light of a wavelength of 664 nm, so as to emit a visible fluorescence, and upon its transition to the ground state, it generates active oxygen, thereby exerting the cell-killing effect on the macrophages.

In particular, the talaporfin-gold complex of formula (II) or its salt has a property such that gold complex (II) or its salt having received the irradiation of an X-ray beam containing an X-ray of a wavelength of 0.153 Å is excited by absorption of the X-ray of said wavelength, and that upon its transition to the ground state, the gold complex of formula (II) gives energy to other electrons within the gold atoms to release the electrons (so-called Auger effect), thereby exhibiting the cell-killing effect on the macrophages.

When the compound of formula (I) above or the compound of formula (II) above is administered in the diagnostic method or the therapeutic method according to this invention, the dose of the compound of formula (I) or (II) administered may vary depending on the method of administration or the object to be achieved. In the case of systemic administration of the compound via a vein, the compound of formula (I) or the compound of formula (II) may be administered at a dose of 0.1 mg/kg to 5 mg/kg, more preferably 0.2 mg/kg to 2 mg/kg.

In the case of topical administration, it is appropriate to administer at a proper dosage such compound that can afford a fluorescence image or an X-ray absorption image within the Langerhans islet inflammatory sites present in the pancreas having IDDM, with the afforded image being observable in a high contrast. To this end, for example, it is suitable to use a method of injecting, via a pancreatic duct, few ml of a solution in which the compound of formula (I) or the compound of formula (II) is dissolved at a concentration of 0.1 to 1 mg/kg in a volume of physiological saline.

The compound of formula (I) and the compound of formula (II) described above are evidently non-toxic, as long as they are administered at a dose usually used in the diagnosis or therapy according to this invention. For example, even when talaporfin, namely the compound of formula (I) was administered at a dose of up to 20 mg/kg, none of significantly toxic observations was found in experimental animals.

In addition, a benzoporphyrin derivative such as verteporfin, etiopurpurin (Purlytin: trade name), Lu-tex, ATX-S10, temoporfin (Foscan; trade name) or Photofrin (trade name) is suitable as the photosensitizers to be used in the invention. These photosensitizers can be used in a free from or in the form of a pharmaceutically acceptable salt thereof or a solvate thereof in the invention. The benzoporphyrin derivative is administered at a dose of 0.1 to 0.5 mg/kg and may be irradiated with a laser light of a wavelength of 690 nm. Etiopurpurin is administered at a dose of 0.2 to 1 mg/kg and may be irradiated with a laser light of a wavelength of 665 nm, and Lu-tex is administered at a dose of 1

to 3 mg/kg and may be irradiated with a laser light of a wavelength of about 730 nm. ATX-S10 or Foscan is administered at a dose of 0.1 to 0.5 mg/kg and may be irradiated with a laser light of a wavelength of 652 nm. Photofrin is administered at a dose of 1 to 3 mg/kg and may be irradiated with a laser light of a wavelength of 630 nm.

When the photosensitizer, for example, the compound of formula (I) is administered, the compound of formula (I) after the administration thereof can be uptaken specifically and selectively by the macrophages having clustered in the Langerhans islet inflammatory sites with IDDM where the macrophages acting on the pancreatic β cell have infiltrated, so that the compound of formula (I) can accumulate in the said inflammatory lesion sites. When a suitable and sufficient period of time is allowed to elapse from the time of administration of the compound, for instance, when a time of few minutes to 48 hours, preferably a time of few minutes to 24 hours is allowed to elapse in the case of intravascular administration of the compound of formula (I), there can elapse a period of time which is sufficient to permit the compound of formula (I) to be uptaken specifically by the macrophage having clustered in the inflammatory sites of Langerhans islets. After the elapse of such sufficient time period, the inflammatory sites of Langerhans islets or a part of the pancreas containing said inflammatory sites are or is then irradiated with a light ray of a specific wavelength, preferably a laser light, by means of an optical fiber which has been inserted via the skin and via a pancreatic duct into said inflammatory sites or into a blood vessel near to said inflammatory sites, or by means of a laparoscope or by means of an endoscope inserted into the pancreatic duct, or by means of an endoscope inserted in the gastric cavity or duodenal cavity. The irradiation of the light ray to be applied to the compound of formula (I) should necessarily be made at a light irradiance which can induce emission of a visible fluorescence from the compound (I) so as to permit visible observation of a fluorescence image. With the compound of formula (I), red laser lights containing a light of a wavelength of 664 nm may usually be irradiated at an irradiance of 20 to 200 $J/cm^2$.

Further, when an X-ray absorbing substance such as the compound of formula (II) is administered, it is necessary that the X-ray to be irradiated should be applied at an irradiance such that the X-ray beam having passed through the body of patient can be observed by the X-ray radiography with taking a photography of an X-ray absorption image on an X-ray film or in another X-ray photography unit. When the compound of formula (II) is administered, an X-ray beam containing an X-ray of a wavelength of 0.153 Å may be applied at an energy of 5 to 50 keV.

Endoscopic retrograde cholangiopancreatography (ERCP) can be used for irradiation of the inflammatory sites of Langerhans islets with light rays, and then an ERCP unit is used for the irradiation of laser lights. The inflammatory sites of Langerhans islets with IDDM as irradiated with light rays can emit a fluorescence selectively, and thus the IDDM lesion sites, which are emitting this fluorescence, can be observed directly with naked eyes by means of an inserted optical fiber scope or on a CRT screen scope.

For irradiating the inflammatory sites of Langerhans islets with laser lights, the photosensitizer is administered as an effective agent and then the inflammatory sites of Langerhans islets are irradiated with laser lights delivered from the top of a quartz fiber which has been inserted in the vicinity of the inflammatory sites of Langerhans islets. Because the pancreas is close to the pylorus in a lower part of the stomach and is close to the duodenum, the pancreas can be irradiated with laser lights delivered from an endoscope which is equipped with a laser light-irradiating optical fiber and which has been inserted into the gastric cavity or duodenal cavity.

On the other hand, when the X-ray absorbing compound of formula (II) is administered, an X-ray beam containing an X-ray of a wavelength of 0.153 Å is to be applied as described above, and the X-ray beam containing the X-ray of a wavelength of 0.153 A is available by using a known X-ray generator. However, when a coherent X-ray is to be used, a large-scale radiation facility, SPring-8, in Harima Science Garden City, Japan or radiation facilities in High Energy Accelerator Research Organization in Tsukuba City, Japan can be utilized.

In order to ensure that the X-ray absorbing substance, for example the compound of formula (II), has certainly been uptaken by and into the infiltrating macrophages after the administration of the compound of formula (II) to the body of a mammal with IDDM, it is necessary to allow a sufficient time of few minutes to 48 hours, preferably few minutes to 24 hours, to elapse from the time of administration. After the elapse of such sufficient time, the inflammatory sites of Langerhans islets and the pancreas or a part thereof containing said inflammatory sites are then selectively irradiated with X-ray beam. The X-ray beam is applied usually from the outside of the body of patient. The X-ray having passed through the pancreas and through the body of the patient is received on an X-ray film to take a photography of an X-ray absorption image thereon, whereby the inflammatory sites of Langerhans islets can be observed as a photography of the X-ray absorption image produced in said inflammatory sites. With the resultant photographed X-ray absorption image, IDDM and pancreatic insulitis can be detected or diagnosed. Further, when the irradiance of X-ray is increased, the excited X-ray absorbing substance is able to damage or destroy the infiltrating macrophages having uptaken the X-ray absorbing substance therein, by the action of the X-ray irradiation or by the electron-releasing function, that is, the Auger effect in the case of administration of the compound of formula (II). Thereby, the pancreatic islet β cells can be protected from the macrophages and thus a therapeutic treatment of IDDM and pancreatic insulitis can be effected.

In the therapy of pancreatic insulitis and IDDM with using the therapeutic method according to the invention, the present inventors have now devised two procedures, one is a therapeutic process of making the irradiation of laser

lights delivered from a fiber catheter as inserted into the inflammatory sites of Langerhans islets, and the other is a therapeutic process of making the irradiation of X-ray beam from the outside of the patient body. In the first procedure comprising the insertion of a fiber catheter into the inflammatory sites of Langerhans islets, the pancreatic β cells are directly irradiated with laser lights, and a fluorescence unique to the administered photosensitizer can be observed, whereby the positions of the inflammatory sites of Langerhans islets can be detected and decided accurately, and thus a direct therapy of the inflammatory sites of Langerhans islets can be conducted accurately and topically. In the second procedure of making the irradiation of X-ray, the inflammatory sites of Langerhans islets are irradiated with X-ray delivered externally from the outside of the patient body and an X-ray absorption occurring in the pancreas can be observed by the X-ray radiography, whereby the positions of the Langerhans islets inflammatory sites can be detected and grasped, and so a therapy of pancreatic insulitis can be conducted directly, accurately and topically in a non-invasive manner.

Brief Description of the Drawings

Figure 1 is a graph which shows an evidently prolonged survival curve of plotting the survival rate (%) of a PDT treated group of BB rats having received a PDT method comprising the administration of talaporfin tetrasodium salt and the irradiation of a laser light after the onset of IDDM in the BB rats in Test Example 3 as described hereinafter, in comparison with a survival curve of plotting the survival rate (%) of the control group (untreated) of the BB rats after the onset of IDDM in Test Example 3.

Figure 2 is a graph which shows an evidently improved survival curve of plotting the survival rate (%) of a PDT treated group of BB rats having received a PDT method comprising the administration of talaporfin tetrasodium salt and the irradiation of a laser light after the onset of IDDM in the BB rats in Test Example 5 as described hereinafter, in comparison with a survival curve of plotting the survival rate (%) of a comparative group of the BB rats (positive control) having received the administration of talaporfin tetrasodium salt but no irradiation of a laser light in Test Example 5 described hereinafter.

Hereinafter, this invention is illustrated in more detail with reference to Test Examples below, but this invention is not limited to these Examples.

Test Example 1

BB/W rats, which had exhibited a morbid state of IDDM morphologically and biochemically similar to human IDDM, were used as test animals. From 60 days after birth, the BB/W rats were examined every day for the urine sugar level and blood sugar level by using GLUTEST SENSOR (Sanwa Kagaku Co. Ltd., Japan). To those BB/W rats whose urine sugar became positive to confirm the onset of IDDM was administered tetrasodium salt of talaporfin of formula (I) at a dose of 5 mg/kg via a tail vein. One hour after the administration, the abdomens of the rats were opened under ether anesthesia and their pancreases were excised. The excised pancreases were frozen in dry ice/acetone, to prepare frozen pancreas sections of 5 μm in thickness. The frozen pancreas sections were irradiated with a semiconductor laser light of 664 nm delivered from a laser generator (Matsushita Industrial Equipment Co., Ltd., Japan). An image of a red fluorescence emitted by talaporfin in the frozen pancreas section was observed under a fluorescence microscope equipped with a notched filter for cutting 664 nm wavelength (made by Carl Zeiss Co., Ltd.) Further, the frozen pancreas sections were each stained by an immunological histochemical method with a rat macrophage-specific antibody (made by BMA).

As a result, the macrophage antibody-positive and selectively stained macrophages were found to have infiltrated and be present in the Langerhans islets in the test pancreas section, and it was confirmed that in the BB rats, the infiltration of the macrophages in the Langerhans islets took place immediately after the onset of IDDM. Further, the regions of the pancreas section which indicated the previously observed fluorescence image of talaporfin were confirmed to be consistent with the places or sites where the stained images of the antibody-positive macrophages were found to be present in the pancreas sections. Thus, it was estimated that talaporfin administered was uptaken by and accumulated specifically in the macrophages having infiltrated the Langerhans islet inflammatory sites.

When a method comprising irradiation of a UV ray of a wavelength of 405 nm and observation of a fluorescence of 672 nm with a cut filter for cutting light rays of wavelengths of not more than 600 nm was also used for the above-mentioned observation of the fluorescence of talaporfin, there could again be obtained the same result as that obtained in the above-described method comprising irradiation of the 664 nm semiconductor laser.

Test Example 2

A sodium salt of the talaporfin-gold complex of formula (II) was administered in the same manner as in Test Example 1 at a dose of 5 mg/kg via a tail vein into BB rats whose urine sugar became positive with showing a hyperglycemia and in which the onset of IDDM was confirmed. One hour after the administration, the abdomens of the rats were opened under ether anesthesia, and their pancreases were excised. The excised pancreases were fixed with glutaric acid and fixed again and embedded in the probes for observation under an electron microscope. Thereafter, very thin test sections of the pancreas were prepared, and gold atom particles present in the test pancreas sections could be observed in a transmitted state under an electron microscope. As a result, it was confirmed that the talaporfin-gold complex had been uptaken specifically by the macrophages having infiltrated the Langerhans islets inflammatory sites.

It was thus confirmed that, like talaporfin, the talaporfin-gold complex had also been uptaken specifically by and accumulated in the macrophages having infiltrated the Langerhans islets inflammatory sites at an early stage of the onset of IDDM.

Test Example 3

From 60 days after birth, BB rats (16 rats per group) were examined every day for the urine sugar level and blood sugar level, to confirm the time of the onset of IDDM. To those BB rats whose urine sugar became positive to confirm the onset of IDDM was administered via a tail vein talaporfin tetrasodium salt at a dose of 5 mg/kg. Twenty-four hours after the administration, a 664-nm laser light at an irradiance of 50 J/cm$^2$ was applied transcutaneously via the back of rats to the target sites, that is, the Langerhans islets in the pancreas and their surrounding region around the Langerhans islets. Photodynamic therapy of IDDM was thus conducted once, and thereafter, the number of surviving BB/W rats was counted every day and the survival rate (%) of the BB rats were evaluated during the breading of rats for the subsequent 32 days.

The survival rate (%) of rats under test was evaluated by the following equation:

$$\text{Survival rats (\%)} = \frac{\text{Number of surviving rats at the day of measurement}}{\text{Total number of rats under test}} \times 100$$

The number of days after the day of the onset of IDDM and the values of the survival rate (%) of rats at each day of measurement are shown in Table 1 below, in respect of the treated group of rats (n=16) having received the above mentioned PDT method as well as the untreated group of rats (n=17) (a control group having received neither administration of the test drug nor irradiation of the laser light).

Table 1

| Number of days after day of IDDM onset | Survival Rate (%) | |
|---|---|---|
| | PDT treated group | Untreated group (control) |
| 1 | 100 | 100 |
| 2 | 100 | 88.2 |
| 3 | 100 | 82.4 |
| 4 | 100 | 82.4 |
| 5 | 100 | 76.5 |
| 6 | 100 | 70.6 |
| 7 | 93.8 | 64.7 |
| 8 | 81.3 | 47.1 |
| 9 | 75 | 41.1 |
| 10 | 62.5 | 41.1 |
| 11 | 62.5 | 29.4 |
| 12 | 56.3 | 23.5 |
| 13 | 50 | 23.5 |
| 14 | 43.8 | 23.5 |
| 15 | 43.8 | 17.6 |
| 17 | 43.8 | 17.6 |

Table 1 (continued)

| Number of days after day of IDDM onset | Survival Rate (%) | |
|---|---|---|
| | PDT treated group | Untreated group (control) |
| 18 | 43.8 | 11.8 |
| 20 | 37.5 | 11.8 |
| 21 | 37.5 | 5.9 |
| 22 | 37.5 | 0 |
| 26 | 37.5 | 0 |
| 27 | 18.8 | 0 |
| 31 | 18.8 | 0 |
| 32 | 6.3 | 0 |

The survival curves of plotting the survival rate (%) of the rats under test in relation to the number of days after the day of onset of IDDM for the treated group of rats and for the untreated group of rats are shown in Figure 1 of the attached drawings.

From the comparison of the survival curve of the PDT treated group of rats with the survival curve of the untreated group of rats (the control group, n=17), it can be seen that the treated group of rats treated by the above PDT method showed an evident prolongation of their survival curve, as compared with that of the untreated group of rats. Thus, the survival rate (%) of the PDT treated group of rats was 100% on Day 6 after the day of administration of talaporfin, and one rat surviving still after Day 32 was confirmed. On the other hand, the survival rate (%) of the untreated group of rats (control group) was about 70% on Day 6 after the start of the test, and all of the untreated rats had died on Day 22 after the start of the test. These results will be clear from the survival curves shown in Figure 1 of the attached drawings.

As a result of the fact that the macrophages having infiltrated the inflammatory sites of Langerhans islets having pancreatic insulitis were killed by the PDT method comprising the administration of talaporfin and irradiation of laser light, it was clearly demonstrated that the progress of the inflammation of Langerhans islets, that is, the pancreatic insulitis was suppressed by the PDT method and the survival curve of the PDT treated rats was improved.

In the PDT treated group of rats, as compared with the untreated group of rats (control group), the blood sugar level had changed at slightly lower stages as shown by tests of measuring the blood sugar level, and there was observed a tendency that the disappearance of insulin production attributable to IDDM was delayed.

The dose of 5 mg/kg of talaporfin tetrasodium salt with which the effectiveness of the photodynamic therapy done for IDDM in BB/W rats was demonstrated, may correspond to a dose of 0.5 mg/kg to 1.0 mg/kg of talaporfin tetrasodium salt in a human, when estimated from comparison between human and rat in respect of the change of the concentration of talaporfin tetrasodium salt in serum. From this, it is expectable that intravenous injection of talaporfin tetrasodium salt at a dose of 0.5 mg/kg to 2.0 mg/kg is effective for therapeutic treatment of human IDDM and pancreatic insulitis.

Test Example 4

A sodium salt of the talaporfin-gold complex was administered at a dose of 5 mg/kg via a tail vein in the same manner as in Test Example 3 into BB/W rats having a confirmed onset of IDDM, whose urine sugar became positive with showing a hyperglycemia. Twenty-four hours after the administration, a coherent X-ray of 0.153 Å, which is specific to the absorption of X-ray by the electrons in the K shell of the gold atoms in the talaporfin-gold complex, was applied (at SPring-8 radiation facility, at an irradiation energy of 25 keV) transcutaneously from the outside of the rats via the back of the rats to the target sites, that is, the Langerhans islets in the pancreas and their surrounding region. The X-ray having passed through the body of rat was received on an X-ray film (Fuji IX25) and the photographed X-ray absorption image in the X-ray film was observed, revealing that the talaporfin-gold complex accumulated in some regions of the Langerhans islets in the pancreas.

Further, the survival rate (%) of the rats having received the PDT method comprising the administration of talaporfin-gold complex and irradiation of X-ray was evaluated in comparison with that of the untreated group of rats (control group). As a result, it was recognized that, like Test Example 3, the survival curve of the PDT treated group of rats was evidently prolonged or improved as compared with that of the untreated group of rats (control group).

The dose of 5 mg/kg of the talaporfin-gold complex, with which the efficacy of the photodynamic therapy done in BB/W rats was demonstrated, may correspond to a dose of about 0.5 mg/kg to 1.0 mg/kg of said gold complex in a human, when estimated from comparison between human and rat in respect of the changes in the concentration of

the test drug in serum.

Test Example 5

In Test Example 3, it was found that the survival curve of plotting the survival rate (%) of the PDT treated group of rats had a tendency to prolong than that of the untreated group of rats (control). In order to make a more exact comparison, the uterine children born from the same parents of BB/W rats were halved into two equivalent groups of rats, and the first group was allotted to a PDT treated group of rats and the second group was allotted to a comparative, control group of rats which received only administration of the test drug.

Thus, BB/W rats were used as test animal. From 60 days after birth, the urine sugar level and blood sugar level of the rats were measured every day to confirm the day of onset of IDDM. The BB/W rats whose blood sugar level was positive (200 mg/dl or higher) to show a confirmed onset of IDDM were halved into two equivalent groups each composed of the uterine children rats. The first group of rats was allotted to the PDT treated group of rats (n=14) and the second group of rats was allotted to the comparative, control group of rats (n=10) which received administration of the test drug but no irradiation of laser light.

On the day of onset of IDDM, to the PDT treated group of rats and to the comparative, control group of rats was administered talaporfin tetrasodium salt at a dose of 5 mg/kg per rat via a tail vein. In the PDT treated group of rats, at the end of 12 hours after the administration of the test drug, a 664 nm laser light was applied at light irradiance of 50 J/cm$^2$ to the skin surface in the abdomen region of the rats so that the Langerhans islets in the pancreas and their surrounding regions as the irradiated target were irradiated with the laser light transcutaneously. In the control group of rats, no irradiation of laser light was effected. The rats under test were bred for 72 days subsequent to the day of onset of IDDM.

On and after the day of onset of IDDM, that is to say, from the day of administration of the test drug, the numbers of the surviving rats in the PDT treated group and in the control group of rats were counted and the values of the survival rate (%) of rats were evaluated. The relationship of the survival rate (%) of rats to the number of days after the day of onset of IDDM is tabulated in Table 2 below. Besides, the evaluation of the survival rate (%) of rats was made by using a known Kaplan-Meier method.

Table 2

| Number of days after day of IDDM onset | Survival Rate (%) | |
|---|---|---|
| | PDT treated group | Comparative control group |
| 1 | 100 | 100 |
| 4 | 100 | 100 |
| 6 | 92.6 | 90 |
| 8 | 78.6 | 90 |
| 10 | 78.6 | 90 |
| 11 | 80 | 71.4 |
| 14 | 71.4 | 70 |
| 15 | 71.4 | 50 |
| 16 | 64.3 | 40 |
| 18 | 64.3 | 30 |
| 20 | 64.3 | 30 |
| 21 | 57.1 | 20 |
| 23 | 57.1 | 20 |
| 24 | 50 | 20 |
| 30 | 50 | 20 |
| 42 | 50 | 20 |
| 43 | 50 | 10 |
| 45 | 50 | 10 |
| 46 | 42.9 | 0 |
| 50 | 42.9 | 0 |
| 58 | 42.9 | 0 |
| 59 | 35.7 | 0 |
| 70 | 35.7 | 0 |

Table 2 (continued)

| Number of days after day of IDDM onset | Survival Rate (%) | |
|---|---|---|
| | PDT treated group | Comparative control group |
| 72 | 35.7 | 0 |

The survival curves of plotting the survival rates (%) of rats evaluated in the results of Table 2 above are shown in Figure 2 of the attached drawings. With the survival curves of rats shown in Figure 2, the comparison between the PDT treated group of rats and the comparative control group of rats was made according to the Log-Rank Test to reveal that P-value was 0.0345 and a significant difference was at a significance level of 5%.

After the surviving rats were bred for the subsequent 72 days, some surviving rats of the PDT treated group were sacrificed under anesthesia and anatomied for the pathological study. The pancreas and the pancreatic Langerhans islets were observed by the under mentioned procedure to obtain pathological observations of them. Thus, the Langerhans islets were stained with an anti-insulin antibody by means of a commercially available kit for measurement of insulin (a product of Wako Junyaku Co., Ltd., Japan), whereby the Langerhans islets in the pancreas of the surviving rats of the PDT treated group could be stained by the anti-insulin antibody, indicating and confirming that the production of insulin was effected in the Langerhans islets in the pancreas of the surviving rats.

With the experiments above, it can be demonstrated that the macrophages having infiltrated the inflammatory sites of Langerhans isles in the pancreas of the IDDM rats can be killed by the PDT treatment comprising the administration of talaporfin and irradiation of light, and consequently the insulin-producing β cells can survive, that the progress of pancreatic insulitis can thus be suppressed to inhibit an entire destruction of the pancreatic β cells, and that the progress of IDDM can be suppressed or delayed, and in this respect, IDDM can be treated therapeutically so that the life span of the rats of the PDT treated group can be prolonged.

Example 1

Talaporfin tetrasodium salt (100 mg) was dissolved in 40 ml of sterile, distilled water for injection, and the resulting solution was adjusted to pH 7.4 by addition of hydrochloric acid or an aqueous sodium hydroxide as a pH adjustor. The entire volume of the solution so obtained was placed in vials and then lyophilized in a usual manner to afford a lyophilized preparation. Upon use of this preparation, the preparation in each vial was mixed with 4 ml of physiological saline and thereby an injectable solution containing talaporfin tetrasodium salt at a concentration of 25 mg/ml can be prepared.

Example 2

Talaporfin tetrasodium salt (200 mg) was dissolved in physiological saline to a final concentration of 20 mg/ml. This solution can be sterilized to afford an injectable solution for intravenous administration or intramuscular administration.

Industrial Applicability

As described hereinbefore, this invention provides pharmaceutical compositions which contain as an active ingredient a photosensitizer or an X-ray absorbing substance capable of being uptaken specifically by the macrophages having infiltrated the inflammatory sites of Langerhans islets in the pancreas with pancreatic insulitis and which are usable in a PDD method or a PDT method for IDDM and pancreatic insulitis. By conducting a method comprising the administration of a photosensitizer or an X-ray absorbing substance capable of being uptaken by and accumulating in the macrophages having infiltrated the inflammatory sites of Langerhans islets and the subsequent irradiation of a laser light or an X-ray, it is possible to observe a fluorescence or an X-ray absorption which was produced in said inflammatory sites of Langerhans islets in the pancreas with pancreatic insulitis. Further, when irradiance of the irradiated light or X-ray is increased, the photosensitizer or an X-ray absorbing substance present in the infiltrating macrophages can be activated to exert the cell-killing effect on the macrophages and thus to destroy or damage selectively the infiltrating macrophages in the Langerhans islets, whereby destruction and disappearance of pancreatic β cells can be inhibited or delayed and hence the progress of pancreatic insulitis and IDDM can be suppressed. Thus, diagnosis or therapy of IDDM and pancreatic insulitis can be effected by this invention.

## Claims

1. A diagnostic composition for diagnosing insulin-dependent diabetes mellitus and pancreatic insulitis accompanied by destruction of pancreatic β cells, which comprises as an active ingredient a photosensitizer having a property capable of being uptaken specifically by the macrophages having infiltrated the inflammatory sites of Langerhans islets in the pancreas of a mammal with the insulin-dependent diabetes mellitus, as well as a property capable of being activated upon absorption of a light ray of a specific wavelength to emit a visible fluorescence, in combination with a pharmaceutically acceptable carrier for the active ingredient.

2. The composition according to claim 1, wherein the photosensitizer is chlorin e6, a chlorin e6 derivative, a benzoporphyrin derivative, etiopurpurin, Lu-Tex, ATX-S10, Foscan and Photofrin, and a pharmaceutically acceptable salt thereof.

3. The composition according to claim 1, wherein the photosensitizer is talaporfin represented by formula (I):

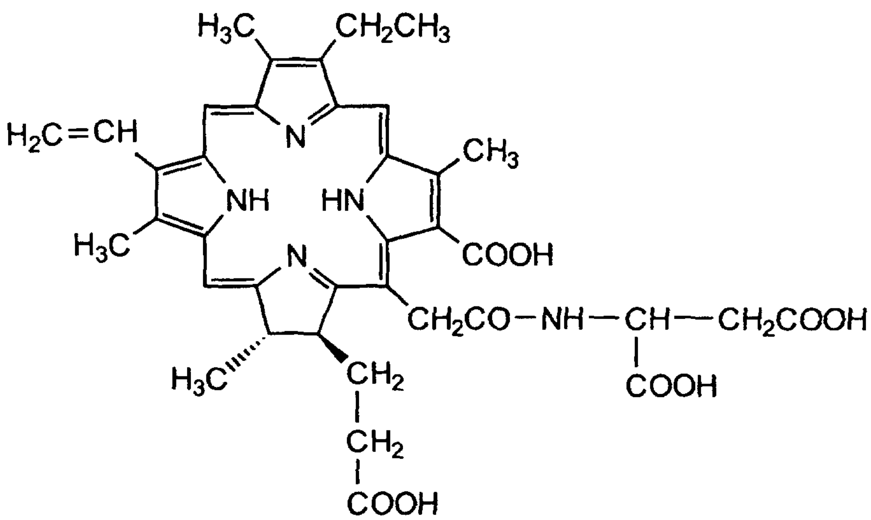

(I)

or a pharmaceutically acceptable salt or a solvate thereof.

4. A method for detecting insulin-dependent diabetes mellitus and pancreatic insulitis, which comprises:

administering to a mammal to be diagnosed an effective amount of a photosensitizer or a pharmaceutical composition containing said photosensitizer as an active ingredient, said photosensitizer having a property capable of being uptaken specifically by the macrophages having infiltrated the inflammatory sites of Langerhans islets in the pancreas of the mammal, as well as a property capable of being activated upon absorption of a light ray of a specific wavelength to emit a visible fluorescence;
irradiating at least a part of the pancreas with a light ray of a specific wavelength suitable for photo-excitation of the administered photosensitizer, at a sufficient irradiance of the irradiated light ray, whereby the visible fluorescence is caused to emit in the pancreas from the photosensitizer present in the macrophages having uptaken said photosensitizer therein;
observing the visible fluorescence emitted in the inflammatory sites of Langerhans islets, and
detecting by such observation the inflammatory sites of Langerhans islets occurring in the pancreas.

5. A method for diagnosing insulin-dependent diabetes mellitus and pancreatic insulitis accompanied by destruction of pancreatic β cells, by the photodynamic diagnosis method, **characterized in that** the method comprises:

administering to a mammal to be diagnosed an effective amount of a photosensitizer or a pharmaceutical composition containing said photosensitizer as an active ingredient, said photosensitizer having a property capable of being uptaken specifically by the macrophages having infiltrated the inflammatory sites of Langerhans islets in the pancreas of the mammal with the insulin-dependent diabetes mellitus, as well as a property capable of being activated upon absorption of a light ray of a specific wavelength to emit a visible fluorescence;

allowing time to elapse for a sufficient time which permits the administered photosensitizer to have been uptaken in the macrophages having infiltrated the inflammatory sites of Langerhans islets in the pancreas of the mammal;

and after the elapse of such sufficient time, irradiating at least a part of the pancreas or the inflammatory sites of Langerhans islets where the infiltrating macrophages having uptaken the administered photosensitizer therein are present, with a selected light ray of a specific wavelength suitable for photo-excitation of the administered photosensitizer, at a sufficient irradiance of the irradiated light ray, whereby the visible fluorescence is caused to emit from said photosensitizer contained in the macrophages present in the inflammatory sites of Langerhans islets infiltrated by said macrophages containing said photosensitizer therein, so that the macrophages present in the inflammatory sites of Langerhans islets can selectively show a fluorescence image;

observing by an endoscope the presence or absence of the inflammatory sites containing the macrophages which show the fluorescence image;

detecting by such observation the presence of the inflammatory sites of Langerhans islets which show the fluorescence image; and

diagnosing by such detection an onset of pancreatic insulitis accompanied by destruction of pancreatic β cells, and an onset of insulin-dependent diabetes mellitus caused by the pancreatic insulitis.

**6.** The method according to claim 4 or 5, wherein the administered photosensitizer is talaporfin of the formula (I) shown in claim 3 or a pharmaceutically acceptable salt thereof, particularly talaporfin tetrasodium salt.

**7.** The method according to claim 4 or 5, wherein the administered photosensitizer is talaporfin tetrasodium salt and this is administered by intravenous injection at a dose of 0.1 mg/kg to 5 mg/kg.

**8.** The method according to claim 4 or 5, wherein the administered photosensitizer is talaporfin tetrasodium salt, and wherein after intravenous injection of talaporfin tetrasodium salt, at least a part of the pancreas or the inflammatory sites of Langerhans islets is or are irradiated with a red-colored laser light containing a light of 664 nm wavelength by means of a laparoscope or by means of an endoscope inserted into the pancreatic duct, or by means of an endoscope inserted in the gastric cavity or duodenal cavity.

**9.** The method according to claim 4 or 5, wherein the visible fluorescence developed selectively from the macrophages present in the inflammatory sites of Langerhans islets is detected by means of a laparoscope placed near to said inflammatory sites or by means of an endoscope inserted in the pancreatic duct.

**10.** Use of talaporfin or a pharmaceutically acceptable salt thereof, as a photosensitizer, in the manufacture of a pharmaceutical product for diagnosing by the photodynamic diagnosis method the insulin-dependent diabetes mellitus and pancreatic insulitis accompanied by destruction of pancreatic β cells.

**11.** A diagnostic composition for diagnosing insulin-dependent diabetes mellitus and pancreatic insulitis accompanied by destruction of pancreatic β cells, **characterized in that** the composition comprises as an active ingredient an X-ray absorbing substance having a property capable of being uptaken specifically by the macrophages having infiltrated the inflammatory sites of Langerhans islets in the pancreas of a mammal with the insulin-dependent diabetes mellitus, as well as a property capable of absorbing an X-ray upon receiving irradiation of the X-ray, in combination with a pharmaceutically acceptable carrier for the active ingredient.

**12.** The composition according to claim 11, wherein the X-ray absorbing substance is a talaporfin-gold complex having the following formula (II):

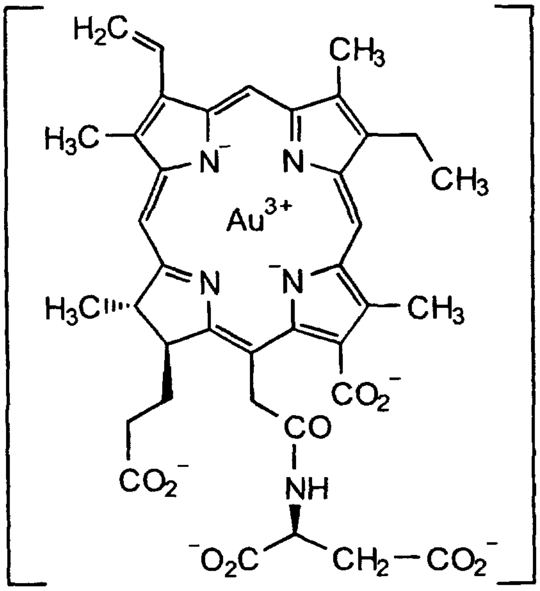

$3H^+$ (II)

or a pharmaceutically acceptable salt or a solvate thereof.

**13.** A method for detecting insulin-dependent diabetes mellitus and pancreatic insulitis, which comprises:

administering to a mammal to be examined an effective amount of an X-ray absorbing substance having a property capable of being uptaken specifically by the macrophages having infiltrated the inflammatory sites of Langerhans islets in the pancreas of a mammal with the insulin-dependent diabetes mellitus, as well as a property capable of absorbing an X-ray upon receiving irradiation of the X-ray;
then irradiating selectively the pancreas of the mammal with an X-ray beam containing an X-ray of a specific wavelength delivered from an X-ray generator placed outside the body of the mammal to be examined;
observing the X-ray absorption occurring in the pancreas, by means of an X-ray radiography; and
detecting by such X-ray radiographic observation the inflammatory sites of Langerhans islets.

**14.** A method for diagnosing insulin-dependent diabetes mellitus and pancreatic insulitis accompanied by destruction of pancreatic β cells, **characterized in that** the method comprises:

administering to a mammal to be diagnosed an effective amount of an X-ray absorbing substance or a pharmaceutical composition containing said X-ray absorbing substance as an active ingredient, said X-ray absorbing substance having a property capable of being uptaken specifically by the macrophages having infiltrated the inflammatory sites of Langerhans islets in the pancreas of a mammal with the insulin-dependent diabetes mellitus, as well as a property capable of absorbing an X-ray upon receiving irradiation of the X-ray;
allowing time to elapse for a sufficient time which permits the administered X-ray absorbing substance to have been uptaken in the macrophages having infiltrated the inflammatory sites of Langerhans islets in the pancreas of the mammal;
and after the elapse of such sufficient time, irradiating selectively the pancreas of the mammal with an X-ray beam containing an X-ray of a specific wavelength delivered from an X-ray generator placed outside the body of the mammal to be examined, at a sufficient irradiance of the irradiated X-ray;
receiving on an X-ray film the X-ray beam having passed through the body of the mammal to take a photography of the X-ray absorption occurring in the pancreas;
observing in the photographed X-ray absorption image of said X-ray film the presence or absence of the X-ray absorption image shown by the group of the macrophages having infiltrated the inflammatory sites of Langerhans islets and having uptaken specifically therein the administered X-ray absorbing substance;
detecting by such X-ray radiographic observation the presence of the inflammatory sites of Langerhans islets, with reference to the photographed X-ray absorption image of the X-ray film; and
diagnosing by such detection of the inflammatory sites of Langerhans islets an onset of pancreatic insulitis accompanied by destruction of pancreatic β cells, and an onset of insulin-dependent diabetes mellitus caused by the pancreatic insulitis.

**15.** The diagnostic method according to claim 13 or 14, wherein the administered X-ray absorbing substance is a talaporfin-gold complex of the formula (II) shown in claim 12 or a pharmaceutically acceptable salt thereof, particularly tetrasodium salt thereof.

**16.** The method according to claim 13 or 14, wherein the administered X-ray absorbing substance is a tetrasodium salt of the talaporfin-gold complex, and this is administered by intravenous injection at a dose of 0.1 mg/kg to 5 mg/kg.

**17.** The method according to claim 13 or 14, wherein the administered X-ray absorbing substance is talaporfin-gold complex tetrasodium salt, and wherein after intravenous injection of the talaporfin-gold complex tetrasodium salt, the pancreas is irradiated with an X-ray beam containing an X-ray of 0.153 Å wavelength delivered externally from the outside of the body of the mammal.

**18.** Use of a talaporfin-gold complex or a pharmaceutically acceptable salt thereof, as an X-ray absorbing substance, in the manufacture of a pharmaceutical product for diagnosing by the photodynamic diagnosis method process the insulin-dependent diabetes mellitus and pancreatic insulitis accompanied by destruction of pancreatic β cells.

**19.** A therapeutic composition for treating therapeutically insulin-dependent diabetes mellitus and pancreatic insulitis accompanied by destruction of pancreatic β cells, which comprises as an active ingredient a photosensitizer having a property capable of being uptaken specifically by the macrophages having infiltrated the inflammatory sites of Langerhans islets in the pancreas of a mammal with the insulin-dependent diabetes mellitus, as well as a property capable of being activated upon absorption of a light ray of a specific wavelength to emit a visible fluorescence, in combination with a pharmaceutically acceptable carrier for the active ingredient.

**20.** The composition according to claim 19, wherein the photosensitizer is chlorin e6, a chlorin e6 derivative, a benzoporphyrin derivative, etiopurpurin, Lu-Tex, ATX-S10, Foscan and Photofrin, and a pharmaceutically acceptable salt thereof.

**21.** The composition according to claim 19, wherein the photosensitizer is talaporfin represented by formula (I) :

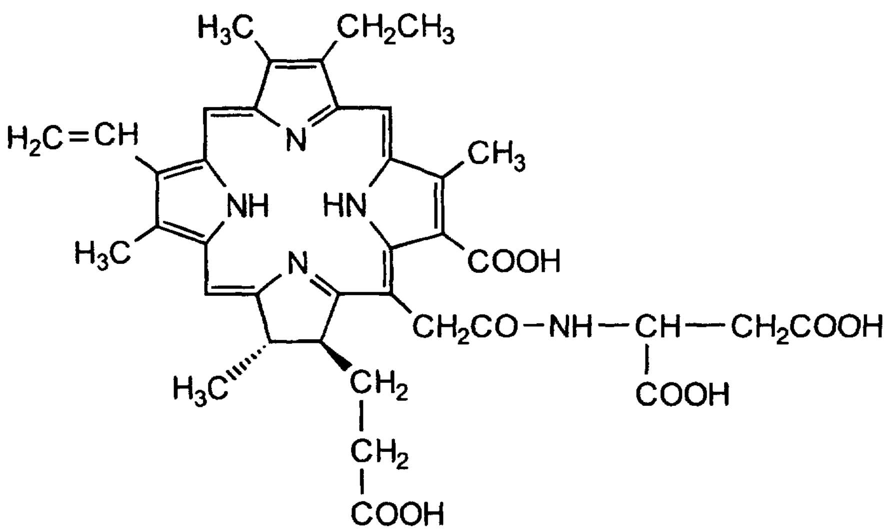

(I)

or a pharmaceutically acceptable salt or a solvate thereof.

**22.** A method for treating therapeutically insulin-dependent diabetes mellitus and pancreatic insulitis accompanied by destruction of pancreatic β cells, by the photodynamic therapy method, **characterized in that** the method comprises:

administering to a mammal to be treated an effective amount of a photosensitizer or a pharmaceutical composition containing said photosensitizer as an active ingredient, said photosensitizer having a property capable

of being uptaken specifically by the macrophages having infiltrated the inflammatory sites of Langerhans islets in the pancreas of the mammal with the insulin-dependent diabetes mellitus, as well as a property capable of being activated upon absorption of a light ray of a specific wavelength to emit a visible fluorescence;
allowing time to elapse for a sufficient time which permits the administered photosensitizer to have been uptaken in the macrophages having infiltrated the inflammatory sites of Langerhans islets in the pancreas of the mammal;
and after the elapse of such sufficient time, irradiating at least a part of the pancreas or the inflammatory sites of Langerhans islets where the infiltrating macrophages having uptaken the administered photosensitizer therein are present, with a selected light ray of a specific wavelength suitable for photo-excitation of the administered photosensitizer, with the irradiation of the selected light ray being effected at an irradiance of the irradiated light ray sufficient to permit that the photosensitizer uptaken and accumulated in the macrophages having infiltrated the inflammatory sites of Langerhans islets is activated by the absorption of the irradiated light ray to generate active oxygen and to damage or destroy said macrophages by active oxygen;
damaging or destroying by such light irradiation the macrophages which infiltrate the inflammatory sites of Langerhans islets; and
inhibiting by such damage or destroy of the macrophages the destruction of the pancreatic β cells caused by initial infiltration of the macrophages and subsequent infiltration of the various inflammatory cells, whereby to suppress or treat therapeutically the pancreatic insulitis and the insulin-dependent diabetes mellitus caused by the pancreatic insulitis.

**23.** The therapeutic method according to claim 22, wherein the administered photosensitizer is talapoporfin or a pharmaceutically acceptable salt thereof, particularly talaporfin tetrasodium salt.

**24.** The therapeutic method according to claim 22, wherein the administered photosensitizer is talaporfin tetrasodium salt and this is administered by intravenous injection at a dose of 0.1 mg/kg to 5 mg/kg.

**25.** The therapeutic method according to claim 22, wherein the administered photosensitizer is talaporfin tetrasodium salt, and wherein after intravenous injection of talaporfin tetrasodium salt, at least a part of the pancreas or the inflammatory sites of Langerhans islets is or are irradiated with a red-colored laser light containing a light of 664 nm wavelength by means of a laparoscope or by means of an endoscope inserted into the pancreatic duct, or by means of an endoscope inserted in the gastric cavity or duodenal cavity, at a light irradiance of 20 to 200 $J/cm^2$.

**26.** Use of talaporfin or a pharmaceutically acceptable salt thereof, as a photosensitizer, in the manufacture of a pharmaceutical product for treating by the photodynamic therapy method the insulin-dependent diabetes mellitus and pancreatic insulitis accompanied by destruction of pancreatic β cells.

**27.** A therapeutic composition for treating therapeutically insulin-dependent diabetes mellitus and pancreatic insulitis accompanied by destruction of pancreatic β cells, **characterized in that** the composition comprises as an active ingredient an X-ray absorbing substance having a property capable of being uptaken specifically by the macrophages having infiltrated the inflammatory sites of Langerhans islets in the pancreas of a mammal with the insulin-dependent diabetes mellitus, as well as a property capable of absorbing an X-ray upon receiving irradiation of the X-ray and capable of being activated by such X-ray absorption to display the electron-releasing function that can damage or destroy the macrophages, in combination with a pharmaceutically acceptable carrier for the active ingredient.

**28.** The composition according to claim 27, wherein the X-ray absorbing substance is a talaporfin-gold complex having the following formula (II):

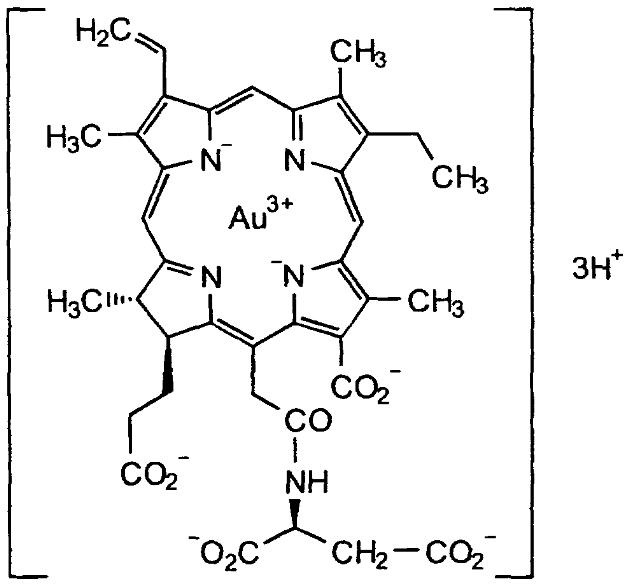

(II)

or a pharmaceutically acceptable salt or a solvate thereof.

**29.** A method for treating therapeutically insulin-dependent diabetes mellitus and pancreatic insulitis accompanied by destruction of pancreatic β cells, by the photodynamic therapy method, **characterized in that** the method comprises:

administering to a mammal to be treated an effective amount of an X-ray absorbing substance or a pharmaceutical composition containing said X-ray absorbing substance as an active ingredient, said X-ray absorbing substance having a property capable of being uptaken specifically by the macrophages having infiltrated the inflammatory sites of Langerhans islets in the pancreas of a mammal with the insulin-dependent diabetes mellitus, as well as a property capable of absorbing an X-ray upon receiving irradiation of the X-ray and capable of being activated by such X-ray absorption to display the electron-releasing function that can damage or destroy the macrophages;
allowing time to elapse for a sufficient time which permits the administered X-ray absorbing substance to have been uptaken in the macrophages having infiltrated the inflammatory sites of Langerhans islets in the pancreas of the mammal;
and after the elapse of such sufficient time, irradiating selectively the pancreas of the mammal with an X-ray beam containing an X-ray of a specific wavelength delivered from an X-ray generator placed outside the body of the mammal to be treated, at a sufficient irradiance of the irradiated X-ray, with the irradiation of the X-ray being effected at an adjusted irradiance of the irradiated X-ray sufficient to permit that the X-ray absorbing substance uptaken and accumulated in the macrophages having infiltrated the inflammatory sites of Langerhans islets is activated by the absorption of the irradiated X-ray to display the electron-releasing function that can damage or destroy the macrophages;
damaging or destroying by such X-ray irradiation the macrophages which infiltrate the inflammatory sites of Langerhans islets; and
inhibiting by such damage or destroy of the macrophages the destruction of the pancreatic β cells caused by initial infiltration of the macrophages and subsequent infiltration of the various inflammatory cells, whereby to suppress or treat therapeutically the pancreatic insulitis and the insulin-dependent diabetes mellitus caused by the pancreatic insulitis.

**30.** The therapeutic method according to claim 29, wherein the administered X-ray absorbing substance is a talaporfin-gold complex of the formula (II) shown in claim 28 or a pharmaceutically acceptable salt thereof, particularly tetrasodium salt thereof.

**31.** The therapeutic method according to claim 29, wherein the administered X-ray absorbing substance is a tetrasodium salt of the talaporfin-gold complex, and this is administered by intravenous injection at a dose of 0.1 mg/kg to 5 mg/kg.

**32.** The therapeutic method according to claim 29, wherein the administered X-ray absorbing substance is talaporfin-gold complex tetrasodium salt, and wherein after intravenous injection of the talaporfin-gold complex tetrasodium salt, the pancreas is irradiated with an X-ray beam containing an X-ray of 0.153 Å wavelength delivered externally from outside of the body of the mammal, at an energy of 5 keV to 50 keV of the irradiated X-ray.

**33.** Use of a talaporfin-gold complex or a pharmaceutically acceptable salt thereof as an X-ray absorbing substance, in the manufacture of a pharmaceutical product for treating therapeutically by the photodynamic therapy method the insulin-dependent diabetes mellitus and pancreatic insulitis accompanied by destruction of pancreatic β cells.

Figure 1

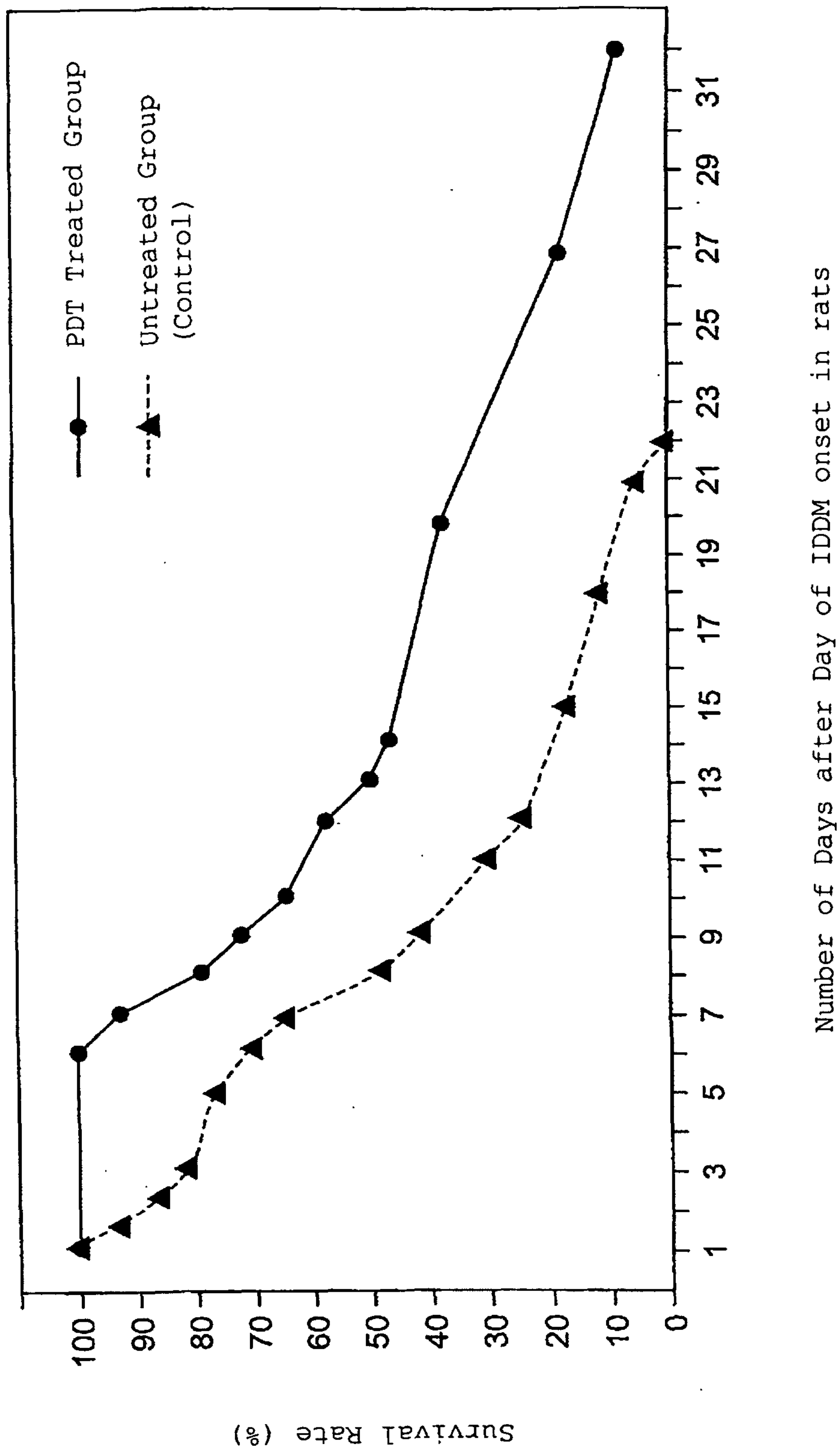
PDT Treated Group
Untreated Group
(Control)
Survival Rate (%)
Number of Days after Day of IDDM onset in rats
100
90
80
70
60
50
40
30
20
10
0
1
3
5
7
9
11
13
15
17
19
21
23
25
27
29
31

Figure 2

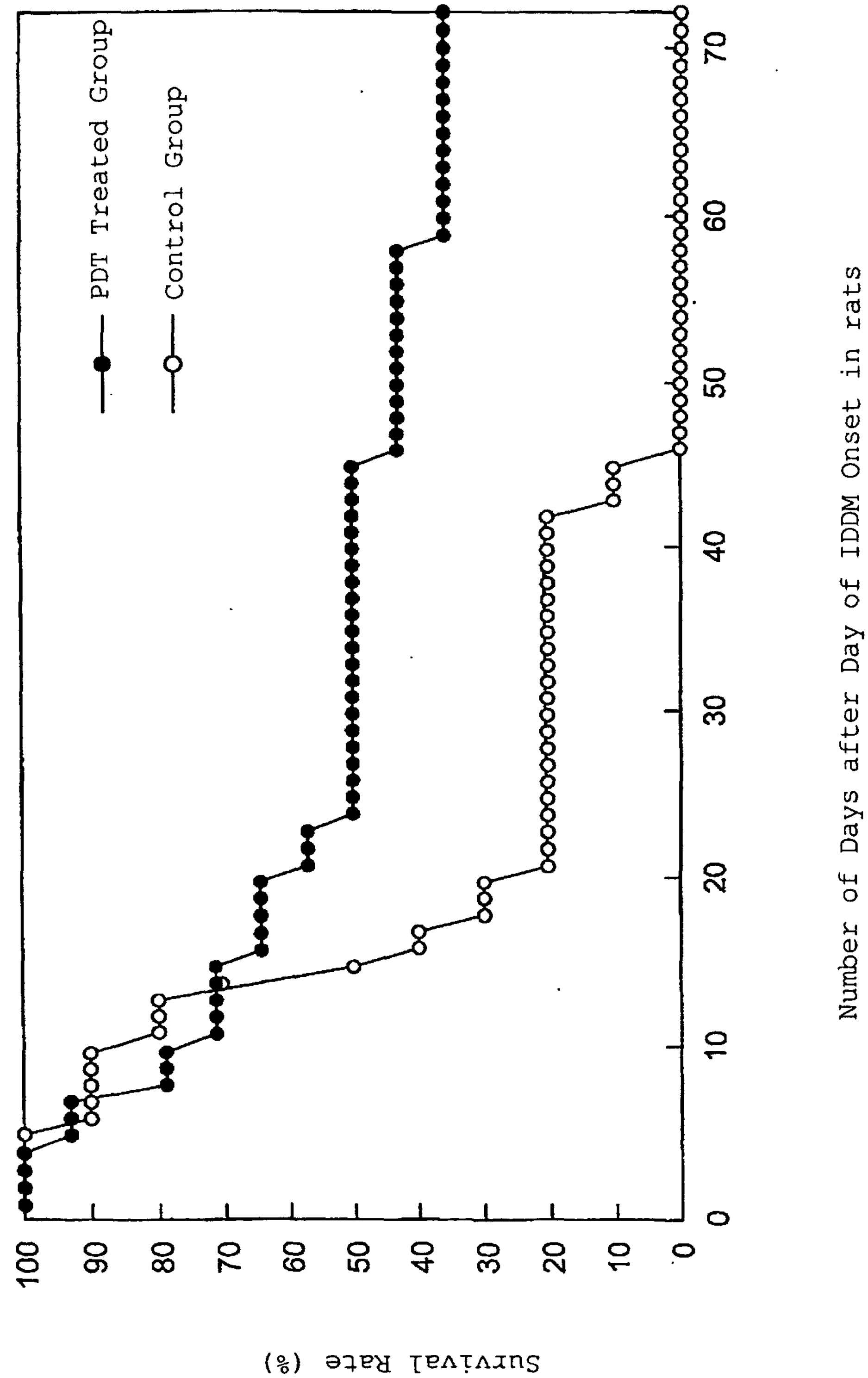
PDT Treated Group
Control Group
Survival Rate (%)
100
90
80
70
60
50
40
30
20
10
0
0
10
20
30
40
50
60
70
Number of Days after Day of IDDM Onset in rats

## INTERNATIONAL SEARCH REPORT

International application No.
PCT/JP02/06510

**A. CLASSIFICATION OF SUBJECT MATTER**

Int.Cl[7] A61K41/00, 31/409, 31/555, 49/00, 49/04, A61P1/18, 3/10, 43/00

According to International Patent Classification (IPC) or to both national classification and IPC

**B. FIELDS SEARCHED**

Minimum documentation searched (classification system followed by classification symbols)

Int.Cl[7] A61K41/00, 31/409, 31/555, 49/00, 49/04, A61P1/18, 3/10, 43/00

Documentation searched other than minimum documentation to the extent that such documents are included in the fields searched

Electronic data base consulted during the international search (name of data base and, where practicable, search terms used)

CA(STN)

**C. DOCUMENTS CONSIDERED TO BE RELEVANT**

| Category* | Citation of document, with indication, where appropriate, of the relevant passages | Relevant to claim No. |
|---|---|---|
| P,X | WO 02/053138 A2 (SHANAHAN-PRENDERGAST, Elisabeth), 02 January, 2002 (02.01.02), Full text; particularly, Claims 28, 31 (Family: none) | 19-21,26-28, 33 |
| X<br>Y | WO 00/01414 A1 (Pharmacyclics Inc.), 13 January, 2000 (13.01.00), Full text & AU 9948608 A & NO 200100044 A & EP 1094840 A1 & KR 2001079498 A & JP 2002-519390 A | 1,11,19,27<br>2,3,10,12, 18,20,21,26, 28,33 |

[x] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03 September, 2002 (03.09.02) | 17 September, 2002 (17.09.02) |
| Name and mailing address of the ISA/ Japanese Patent Office | Authorized officer |
| Facsimile No. | Telephone No. |

Form PCT/ISA/210 (second sheet) (July 1998)

INTERNATIONAL SEARCH REPORT

International application No.
PCT/JP02/06510

C (Continuation). DOCUMENTS CONSIDERED TO BE RELEVANT

| Category* | Citation of document, with indication, where appropriate, of the relevant passages | Relevant to claim No. |
|---|---|---|
| Y | EP 168831 B1 (Nippon Petrochemicals Co., Ltd., Japan),<br>18 September, 1991 (18.09.91),<br>Full text<br>& US 4675338 A & US 4693885 A<br>& DK 167769 B1 & NO 169291 B<br>& AU 592058 B2 & CA 1264740 A1<br>& JP 62-005986 A2 | 2,3,10,12,<br>18,20,21,26,<br>28,33 |

Form PCT/ISA/210 (continuation of second sheet) (July 1998)

## INTERNATIONAL SEARCH REPORT

International application No.
PCT/JP02/06510

**Box I Observations where certain claims were found unsearchable (Continuation of item 1 of first sheet)**

This international search report has not been established in respect of certain claims under Article 17(2)(a) for the following reasons:

1. [X] Claims Nos.: 4-9, 13-17, 22-25, 29-32
   because they relate to subject matter not required to be searched by this Authority, namely:
   Claims 4 to 9, 13 to 17, 22 to 25 and 29 to 32 pertain to methods for treatment of human body by therapy or to methods for diagnosis of human body and thus relate to a subject matter which this International Searching Authority is not required, under the provisions (continued to extra sheet)
2. [ ] Claims Nos.:
   because they relate to parts of the international application that do not comply with the prescribed requirements to such an extent that no meaningful international search can be carried out, specifically:
3. [ ] Claims Nos.:
   because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:
4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)

INTERNATIONAL SEARCH REPORT

International application No.
PCT/JP02/06510

Continuation of Box No.I-1 of continuation of first sheet(1)

of Article 17(2)(a)(i) of the PCT and Rule 39.1(iv)of the Regulations under the PCT, to search.

Form PCT/ISA/210 (extra sheet) (July 1998)